# EUROPEAN PATENT SPECIFICATION

(11) **EP 2 515 110 B1**
(45) Date of publication and mention of the grant of the patent: **25.03.2015**
(21) Application number: 10831343.8
(22) Date of filing: 19.11.2010
(51) Int. Cl.: G01N 33/533, G01N 21/78, G01N 33/531, C07K 16/18

(54) **Fluoroimmunoassay method**
Fluorimmunassay-Verfahren
Procédé de dosage fluoroimmunologique

(30) Priority: 19.11.2009 JP 2009264420
(43) Date of publication of application: 24.10.2012
(73) Proprietor: Ushio Denki Kabushiki Kaisha, Tokyo-to (JP)
(72) Inventor: UEDA, Hiroshi, Tokyo 113-8656 (JP); ABE, Ryoji, Chiba-shi Chiba 260-0856 (JP); IHARA, Masaki, Kamiina-gun Nagano 399-4511 (JP); TAKAGI, Hiroaki, Chiba-shi Chiba 260-0856 (JP)
(74) Representative: Harding, Charles Thomas
(86) International application number: PCT/JP2010/006809
(87) International publication number: WO 2011/061944

(56) References cited:
- EP-A1- 2 062 974
- WO-A1-2006/033413
- WO-A2-2008/051762
- JP-A- 10 078 436
- JP-A- 10 319 017
- JP-A- 2006 506 634
- JP-A- 2007 526 750
- JP-A- 2008 187 936
- RENARD M ET AL: "Knowledge-based Design of Reagentless Fluorescent Biosensors from Recombinant Antibodies", JOURNAL OF MOLECULAR BIOLOGY, ACADEMIC PRESS, UNITED KINGDOM, vol. 318, no. 2, 26 April 2002 (2002-04-26), pages 429-442, XP004449534, ISSN: 0022-2836, DOI: 10.1016/S0022-2836(02)00023-2
- JESPERS L ET AL: "Selection of optical biosensors from chemisynthetic antibody libraries", PROTEIN ENGINEERING, DESIGN AND SELECTION, OXFORD JOURNAL, LONDON, GB, vol. 17, no. 10, 1 October 2004 (2004-10-01), pages 709-713, XP002535021, ISSN: 1741-0126, DOI: 10.1093/PROTEIN/GZH083 [retrieved on 2004-11-10]
- RENARD M ET AL: "Deriving Topological Constraints from Functional Data for the Design of Reagentless Fluorescent Immunosensors", JOURNAL OF MOLECULAR BIOLOGY, ACADEMIC PRESS, UNITED KINGDOM, vol. 326, no. 1, 7 February 2003 (2003-02-07), pages 167-175, XP004450025, ISSN: 0022-2836, DOI: 10.1016/S0022-2836(02)01334-7
- NICOLE MARMÉ ET AL: "Sensitive bioanalysis-combining single-molecule spectroscopy with mono-labeled self-quenching probes", ANALYTICAL AND BIOANALYTICAL CHEMISTRY, SPRINGER, BERLIN, DE, vol. 388, no. 5-6, 12 June 2007 (2007-06-12), pages 1075-1085, XP019537543, ISSN: 1618-2650, DOI: 10.1007/S00216-007-1365-1
- ISSEI IIJIMA ET AL: "Position-Specific Incorporation of Fluorescent Non-natural Amino Acids into Maltose-Binding Protein for Detection of Ligand Binding by FRET and Fluorescence Quenching", CHEMBIOCHEM, vol. 10, no. 6, 17 April 2009 (2009-04-17) , pages 999-1006, XP055057290, ISSN: 1439-4227, DOI: 10.1002/cbic.200800703
- RYOJI ABE ET AL: ""Quenchbodies": Quench-Based Antibody Probes That Show Antigen-Dependent Fluorescence", JOURNAL OF THE AMERICAN CHEMICAL SOCIETY, vol. 133, no. 43, 2 November 2011 (2011-11-02), pages 17386-17394, XP055057266, ISSN: 0002-7863, DOI: 10.1021/ja205925j
- SHEAN-LEE LIM ET AL.: 'Noncompetitive Detection of Low Molecular Weight Peptides by Open Sandwich Immunoassay' ANALYTICAL CHEMISTRY vol. 79, no. 16, 2007, pages 6193 - 6200, XP008156568
- TOMOICHI YOKOZEKI ET AL.: 'A Homogeneous Noncompetitive Immunoassay for the Detection of Small Haptens' ANALYTICAL CHEMISTRY vol. 74, no. 11, 2002, pages 2500 - 2504, XP008156580

### SEQUENCE LISTING

<110> ProteinExpress Co., Ltd.
<120> Fluoroimmunoassay
<130> 2010C6031
<150> JP 2009-264420
   <151> 2009-11-19
<160> 17
<170> PatentIn version 3.1
<210> 1
   <211> 116
   <212> PRT
   <213> Artificial
<220>
   <223> Inventor: Ueda, Hiroshi; Abe, Ryoji Inventor: Ihara, Masaki; Takagi, Hiroaki
<220>
   <223> anti-BGP antibody VH
<400> 1
   Gln Val Lys Leu Gln Gln Ser Gly Ala Glu Phe Val Lys Ala Gly Ala
   1               5                   10                  15
   Ser Val Lys Leu Ser Cys Lys Thr Ser Gly Tyr Thr Phe Asn Asn Tyr
               20                  25                  30
   Trp Ile His Trp Val Lys Gln Ser Pro Gly Gln Gly Leu Glu Trp Ile
           35                  40                  45
   Gly Glu Ile Asp Pro Ser Asp Gly Tyr Ser Asn Tyr Asn Gln Lys Phe
       50                  55                  60
   Lys Gly Lys Ala Thr Leu Thr Val Asp Lys Ser Ser Ser Thr Ala Tyr
   65                  70                  75                  80
   Met His Leu Asn Ser Leu Thr Ser Glu Asp Ser Ala Val Tyr Tyr Cys
                   85                  90                  95
   Thr Ser Ser Thr Ser Val Gly Gly Ser Trp Gly Gln Gly Thr Thr Val
               100                 105                 110
   Thr Val Ser Ser
           115
<210> 2
   <211> 113
   <212> PRT
   <213> Artificial
<220>
   <223> anti-BGP antibody VL
<400> 2
   Asp Ile Glu Leu Thr Gln Ser Pro Leu Ser Leu Pro Val Ser Leu Gly
   1               5                   10                  15
   Asp Gln Ala Ser Ile Ser Cys Thr Ser Ser Gln Ser Leu Leu His Ser
               20                  25                  30
   Asn Gly Asp Thr Tyr Leu His Trp Tyr Leu Gln Lys Pro Gly Gln Ser
           35                  40                  45
   Pro Lys Leu Leu Ile Tyr Thr Leu Ser Asn Arg Phe Ser Gly Val Pro
       50                  55                  60
   Asp Arg Phe Ser Gly Ser Gly Ser Gly Thr Asp Phe Thr Leu Lys Ile
   65                  70                  75                  80
   Ser Arg Val Glu Ala Ala Asp Leu Gly Ile Tyr Phe Cys Ser Gln Thr
                   85                  90                  95
   Thr His Val Pro Tyr Thr Phe Gly Gly Gly Thr Lys Leu Glu Ile Lys
               100                 105                 110
   Arg
<210> 3
   <211> 13
   <212> PRT
   <213> Artificial
<220>
   <223> ProXtag
<220>
   <221> MISC_FEATURE
   <222> (9)..(9)
   <223> X is a fluorescent amino acid.
<400> 3
   Met Ser Lys Gln Ile Glu Val Asn Xaa Ser Asn Glu Thr
   1               5                   10
<210> 4
   <211> 8
   <212> PRT
   <213> Artificial
<220>
   <223> Spacer
<400> 4
   Gly Gly Gly Ser Gly Gly Gly Ser
   1 5
<210> 5
   <211> 21
   <212> PRT
   <213> Artificial
<220>
   <223> Linker
<400> 5
   Leu Val Thr Val Ser Ser Gly Gly Gly Gly Ser Gly Gly Gly Gly Ser
   1 5 10 15
   Gly Gly Gly Gly Ser
   20
<210> 6
   <211> 119
   <212> PRT
   <213> Artificial
<220>
   <223> anti-BPA antibody VH
<400> 6
   Asp Val Gln Leu Gln Glu Ser Gly Pro Gly Leu Val Lys Pro Ser Gln
   1               5                   10                  15
   Ser Leu Ser Leu Thr Cys Ser Val Thr Gly Tyr Ser Ile Thr Ser Gly
               20                  25                  30
   Tyr Tyr Trp Asn Trp Ile Arg Gln Phe Pro Gly Asn Lys Leu Glu Trp
           35                  40                  45
   Met Gly Tyr Ile Arg Tyr Asp Gly Ser Asn Asn Tyr Asn Pro Ser Leu
       50                  55                  60
   Lys Asn Arg Ile Ser Ile Thr Arg Asp Thr Ser Lys Asn Gln Phe Phe
   65                  70                  75                  80
   Leu Lys Leu Asn Ser Val Thr Pro Glu Asp Thr Ala Thr Tyr Tyr Cys
                   85                  90                  95
   Ala Arg Val Leu Gly Arg Gly Tyr Gly Leu Asp Tyr Trp Gly Gln Gly
               100                 105                 110
   Thr Ser Val Thr Val Ser Ser
           115
<210> 7
   <211> 111
   <212> PRT
   <213> Artificial
<220>
   <223> anti-BPA antibody VL
<400> 7
   Asp Ile Val Leu Thr Gln Ser Pro Ala Ser Leu Ala Val Ser Leu Gly
   1 5 10 15
   Gln Arg Ala Thr Ile Ser Cys Arg Ala Ser Gln Ser Val Ser Thr Ser
   20 25 30
   Thr Tyr Ser Tyr Leu His Trp Tyr Gln Gln Arg Pro Gly Gln Pro Pro
   35 40 45
   Lys Leu Ile Lys Tyr Val Ser Asn Leu Glu Ser Gly Val Pro Ala Arg
   50 55 60
   Phe Ser Gly Ser Gly Ser Gly Thr Asp Phe Thr Leu Asn Ile His Pro
   65 70 75 80
   Val Glu Glu Glu Asp Thr Ala Thr Tyr Tyr Cys Gln His Ser Trp Glu
   85 90 95
   Ile Pro Pro Thr Phe Gly Gly Gly Thr Lys Leu Glu Ile Lys Arg
   100 105 110
<210> 8
   <211> 7
   <212> PRT
   <213> Artificial
<220>
   <223> BGP C-terminal peptide
<400> 8
   Arg Arg Phe Tyr Gly Pro Val
   1 5
<210> 9
   <211> 15
   <212> PRT
   <213> Artificial
<220>
   <223> linker
<400> 9
   Gly Gly Gly Gly Ser Gly Gly Gly Gly Ser Gly Gly Gly Gly Ser
   1 5 10 15
<210> 10
   <211> 12
   <212> PRT
   <213> Artificial
<220>
   <223> linker
<400> 10
   Gly Gly Gly Ser Gly Gly Gly Ser Gly Gly Gly Ser
   1 5 10
<210> 11
   <211> 20
   <212> PRT
   <213> Artificial
<220>
   <223> linker
<400> 11
   Gly Gly Gly Ser Gly Gly Gly Ser Gly Gly Gly Ser Gly Gly Gly Ser
   1 5 10 15
   Gly Gly Gly Ser
   20
<210> 12
   <211> 113
   <212> PRT
   <213> Artificial
<220>
   <223> anti-HEL antibody VH
<400> 12
   Glu Val Gln Leu Gln Glu Ser Gly Pro Ser Leu Val Lys Pro Ser Gln
   1 5 10 15
   Thr Leu Ser Leu Thr Cys Ser Val Thr Gly Asp Ser Ile Thr Ser Asp
   20 25 30
   Tyr Trp Ser Trp Ile Arg Lys Phe Pro Gly Asn Arg Leu Glu Tyr Met
   35 40 45
   Gly Tyr Val Ser Tyr Ser Gly Ser Thr Tyr Tyr Asn Pro Ser Leu Lys
   50 55 60
   Ser Arg Ile Ser Ile Thr Arg Asp Thr Ser Lys Asn Gln Tyr Tyr Leu
   65 70 75 80
   Asp Leu Asn Ser Val Thr Thr Glu Asp Thr Ala Thr Tyr Tyr Cys Ala
   85 90 95
   Asn Trp Asp Gly Asp Tyr Trp Gly Gln Gly Thr Leu Val Thr Val Ser
   100 105 110
   Ser
<210> 13
   <211> 108
   <212> PRT
   <213> Artificial
<220>
   <223> anti-HEL antibody-VL
<400> 13
   Asp Ile Val Leu Thr Gln Ser Pro Ala Thr Leu Ser Val Thr Pro Gly
   1 5 10 15
   Asn Ser Val Ser Leu Ser Cys Arg Ala Ser Gln Ser Ile Gly Asn Asn
   20 25 30
   Leu His Trp Tyr Gln Gln Lys Ser His Glu Ser Pro Arg Leu Leu Ile
   35 40 45
   Lys Tyr Ala Ser Gln Ser Ile Ser Gly Ile Pro Ser Arg Phe Ser Gly
   50 55 60
   Ser Gly Ser Gly Thr Asp Phe Thr Leu Ser Ile Asn Ser Val Glu Thr
   65 70 75 80
   Glu Asp Phe Gly Met Tyr Phe Cys Gln Gln Ser Asn Ser Trp Pro Tyr
   85 90 95
   Thr Phe Gly Gly Gly Thr Lys Leu Glu Ile Lys Arg
   100 105
<210> 14
   <211> 116
   <212> PRT
   <213> Artificial
<220>
   <223> anti-Estradiol antibody-VH
<400> 14
   Glu Val Gln Leu Gln Gln Ser Gly Pro Glu Leu Val Lys Pro Gly Ala
   1 5 10 15
   Ser Val Lys Ile Ser Cys Lys Thr Ser Gly Tyr Thr Phe Thr Lys Tyr
   20 25 30
   Thr Ile His Trp Val Lys Gln Thr His Gly Lys Ser Leu Glu Trp Ile
   35 40 45
   Gly Gly Ile Asn Pro Ile Asn Gly Gly Thr Ser Tyr Asn Gln Lys Phe
   50 55 60
   Lys Asp Lys Ala Ser Leu Thr Val Asp Lys Ser Ser Ser Thr Ala Tyr
   65 70 75 80
   Met Glu Leu Arg Ser Leu Thr Ser Lys Asp Ser Ala Val Tyr Tyr Cys
   85 90 95
   Glu Ile Tyr Tyr Gly Ser Phe Asp His Trp Gly Gln Gly Thr Thr Leu
   100 105 110
   Thr Val Ser Ser
   115
<210> 15
   <211> 109
   <212> PRT
   <213> Artificial
<220>
   <223> anti-Estradiol antibody-VL
<400> 15
   Asp Ile Val Leu Thr Gln Ser Pro Ala Ile Met Ser Ala Ala Pro Gly
   1               5                   10                  15
   Glu Lys Val Thr Leu Thr Cys Ser Ala Ser Ser Ser Val Ser Ser Ser
               20                  25                  30
   Lys Leu Phe Trp Tyr Gln Gln Lys Pro Gly Ser Ser Pro Lys Leu Trp
           35                  40                  45
   Ile Tyr Ser Thr Ser Asn Leu Ala Ser Gly Val Pro Ala Arg Phe Ser
       50                  55                  60
   Gly Ser Gly Ser Gly Thr Ser Tyr Ser Leu Thr Ile Ser Asn Met Glu
   65                  70                  75                  80
   Ala Glu Asp Ala Ala Ser Tyr Phe Cys Leu Gln Trp Ser Gly Tyr Pro
                   85                  90                  95
   Pro Thr Phe Gly Ala Gly Thr Lys Leu Glu Ile Lys Arg
               100                 105
<210> 16
   <211> 116
   <212> PRT
   <213> Artificial
<220>
   <223> anti-SA antibody-VH
<400> 16
   Glu Val Gln Leu Leu Glu Ser Gly Gly Gly Leu Val Gln Pro Gly Gly
   1 5 10 15
   Ser Leu Arg Leu Ser Cys Ala Ala Ser Gly Phe Thr Phe Ser Ser Tyr
   20 25 30
   Ala Met Ser Trp Val Arg Gln Ala Pro Gly Lys Gly Leu Glu Trp Val
   35 40 45
   Ser His Ile Ser Pro Tyr Gly Ala Asn Thr Arg Tyr Ala Asp Ser Val
   50 55 60
   Lys Gly Arg Phe Thr Ile Ser Arg Asp Asn Ser Lys Asn Thr Leu Tyr
   65 70 75 80
   Leu Gln Met Asn Ser Leu Arg Ala Glu Asp Thr Ala Val Tyr Tyr Cys
   85 90 95
   Ala Lys Gly Leu Arg Ala Phe Asp Tyr Trp Gly Gln Gly Thr Leu Val
   100 105 110
   Thr Val Ser Ser
   115
<210> 17
   <211> 109
   <212> PRT
   <213> Artificial
<220>
   <223> anti-SA antibody-VL
<400> 17
   Thr Asp Ile Gln Met Thr Gln Ser Pro Ser Ser Leu Ser Ala Ser Val
   1 5 10 15
   Gly Asp Arg Val Thr Ile Thr Cys Arg Ala Ser Gln Ser Ile Ser Ser
   20 25 30
   Tyr Leu Asn Trp Tyr Gln Gln Lys Pro Gly Lys Ala Pro Lys Leu Leu
   35 40 45
   Ile Tyr Arg Ala Ser Leu Leu Gln Ser Gly Val Pro Ser Arg Phe Ser
       50                  55                  60
   Gly Ser Gly Ser Gly Thr Asp Phe Thr Leu Thr Ile Ser Ser Leu Gln
   65                  70                  75                  80
   Pro Glu Asp Phe Ala Thr Tyr Tyr Cys Gln Gln Gly Arg Asp Arg Pro
                   85                  90                  95
   Ala Thr Phe Gly Gln Gly Thr Lys Val Glu Ile Lys Arg
               100                 105

## Description

### Technical field

The present invention relates to a novel antigen-concentration measuring method requiring neither a solid-phase immobilization step nor a washing step, and a kit and the like for carrying out the antigen-concentration measuring method.

### Background Art

Of methods for measuring-concentrations of antigens and antibodies, a most widely used measuring method in clinical diagnosis, fundamental studies and environmental researches is an immunoassay method called a sandwich ELISA method (or sandwich RIA method), which uses two types of monoclonal antibodies recognizing different epitopes of a same antigen or uses a monoclonal antibody and a polyclonal antibody. The sandwich method will be more specifically described as follows. As a first stage, a mono/polyclonal antibody called as a primary antibody is immobilized to a measurement plate. To the plate, a test sample containing an antigen is supplied and allowed to react for a predetermined time to bind the antibody and the antigen. Then, as a second stage, contaminants bound to the antibody and antigen non-specifically bound to the plate are washed away with a washing liquid. As a third stage, a solution of a secondary antibody previously labeled with a reporter molecule such as an enzyme, a fluorescent dye or a radioisotope is supplied and allowed to react for a predetermined time. In this manner, to the antigen captured by the primary antibody, a labeled secondary antibody is further bound. After completion of the reaction, excessive labeled antibody is washed away with a washing liquid and the amount of reporter molecule bound to the measurement plate is measured based on e.g., enzyme activity, fluorescence or radioisotope. In this manner, the level of an antigen in the test sample is measured.

As described above, in a general sandwich ELISA method, two types of antibodies recognizing different epitopes are required. For example, in the case where e.g., a low-molecular compound, is used as an antigen, it is difficult to prepare a plurality of antibodies recognizing different epitopes. Therefore, Ueda et al. established a highly accurate immunoassay method for a low-molecular compound called an open-sandwich method, using a heavy-chain variable region (VH) and a light-chain variable region (VL) of a single antibody (Patent Documents 1 and 2, Non-patent Documents 1 and 2). This method is an antigen-concentration measuring method, which includes preparing a VH-region polypeptide and a VL-region polypeptide of an antibody specifically recognizing an antigen, labeling one of the polypeptides with a reporter molecule to prepare a labeled polypeptide, immobilizing the other polypeptide to a solid phase to prepare an immobilized polypeptide, bringing an antigen-containing sample and the labeled polypeptide into contact with the immobilized polypeptide, and measuring the amount of reporter molecule of the labeled polypeptide bound to the immobilized polypeptide. Furthermore, as a method for measuring a low-molecular compound, e.g., liquid chromatography is known other than the immunoassay method. However, the liquid chromatography requires a highly accurate measurement apparatus and a large amount of test sample and takes a long time for measurement. In addition, its versatility is low.

Furthermore, as an immunoassay method for measuring the concentration of an antigen using a fluorescent-dye labeled antibody, the following methods are known: an immunoassay method (Non-patent Documents 3 and 4), which includes labeling an antibody and an antigen with different fluorescent dyes, respectively, and using a change in efficiency of fluorescent resonance energy transfer (FRET) caused between the fluorescent dyes as a reference; an immunoassay method, which uses a phenomenon where fluorescence of a fluorescence-labeled antibody previously quenched by adding a quencher to the antibody is increased by introduction of a target detection substance, in short, using a change in efficiency by quenching as a reference; and a fluoroimmunoassay method (Patent Document 3), which measures a decrease of fluorescence intensity caused by aggregation of a measuring target substance with an antibody labeled with a fluorescent dye.

Renaurd M. et al, (Journal of Molecular Biology, Vol. 318, No. 2, 2002, pp429-442) described the possibility of obtaining from any antibody a fluorescent conjugate which responds to the binding of the antigen by a variation of its fluorescence. In addition Renaurd M. *et al,* suggests rules of design for constructing antigen-sensitive fluorescent conjugates from any antibody, even in the absence of structural data.

### Prior Art Documents

### Patent Documents

Patent Document 1: Japanese Unexamined Patent Application Publication No. 10-78436
Patent Document 2: Japanese Patent No. 3784111
Patent Document 3: Japanese Unexamined Patent Application Publication No. 10-282098

### Non-patent Documents

Non-patent Document 1: Hiroshi Ueda, Journal of the Pharmaceutical Society of Japan 27: 71-80 (2007)
Non-patent Document 2: Lim SL, et al., Anal Chem. 79 (16): 6193-200 (2007)
Non-patent Document 3: Iijima I. and Hohsaka T., Chembiochem. 17; 10 (6): 999-1006 (2009)
Non-patent Document 4: Kajihara D, et al., Nat Methods. 3 (11): 923 (2006)

### Summary of the Invention

### Object to be Solved by the Invention

Immunoassay methods known up to present, as described above, all require a step of immobilizing an antibody or an antigen to a solid phase and a washing step of removing a labeled compound non-specifically adsorbed. These steps require intricate work and consume time. In addition, the results vary due to these steps. For these reasons, it has been desired to develop a liquid-phase immunoassay method requiring neither a solid-phase immobilization step nor a washing step. An object of the present invention is to provide an immunoassay method requiring neither a solid-phase immobilization step nor a washing step, enabling quick and simple quantitative measurement of a target substance in a liquid phase and capable of visualizing an antigen.

### Means to Solve the Object

The present inventors first attempted to establish an antibody/antigen binding activity evaluation system using antibody VH and VL labeled with different fluorescent dyes with reference to fluorescent resonance energy transfer (FRET) efficiency. In the FRET measurement, a fluorescent-dye CR110-labeled anti-BGP antibody light-chain region (CR110-VL) and a fluorescent-dye TAMRA-labeled anti-BGP antibody heavy-chain region (TAMRA-VH) were used. The inventors predicted that if an antigen is not present, FRET from CR110 to TAMAR will not occur; whereas, if an antigen is present, VH and VL form a ternary complex with the antigen between them, with the result that FRET from CR110 to TAMRA will occur. Based on such a prediction, the inventors incubated CR110-VL and TAMRA-VH or non-labeled VH, together with BGP antigen peptides in different concentrations and analyzed a change of fluorescence intensity of CR110 in accordance with fluorescence intensity multiple distribution analysis (FIDA). As a result, when CR110-VL was reacted with TAMRA-VH, the fluorescence intensity of CR110 decreased in a BGP antigen peptide concentration-dependent manner. From this, it was confirmed that CR110-VL and TAMRA-VH were bound with the antigen peptide between them to form a complex and FRET from CR110 to TAMRA occurred as predicted.

In contrast, unexpectedly, when CR110-VL was reacted with non-labeled VH, the fluorescence intensity of CR110 increased in a BGP antigen peptide concentration-dependent manner. The present inventors, for the purpose of determining whether the same phenomenon is observed when labeled VH is used, incubated TAMRA-VH and CR110-VL or non-labeled VL together with BGP antigen peptides in different concentrations and analyzed a change in fluorescence intensity of TAMRA. As a result, it was found that also in the case where TAMRA-VH was reacted with non-labeled VL, the fluorescence intensity of TAMRA-VH increased in a BGP antigen peptide concentration-dependent manner. These results were totally beyond expectation. The present inventors postulated that VL and VH may serve as a quencher for a fluorescent dye (CR110, TAMRA) and quench is eliminated only when VH and VL form a complex with an antigen peptide between them, with the result that the fluorescence intensity may increase.

Based on the hypothesis, in attempt to establish a novel assay method (hereinafter sometimes referred also to as "homogenous fluorescence based immunoassay") using the quenching phenomenon, the present inventors reacted TAMRA-VH and BGP peptides in different concentrations in the presence or absence of non-labeled VL and measured the fluorescence intensity. As a result, the fluorescence intensity of TAMRA-VH increased in a BGP peptide concentration-dependent manner in the presence of VL. They analyzed the ratio of fluorescence intensity (+VL/- VL) of TAMRA-VH in the presence of VL to TAMRA-VH in the absence of VL. Based on the results, they found that a highly sensitive homogenous fluorescence based immunoassay could be constructed. Furthermore, the inventors determined that the sensitivity of the "homogenous fluorescence based immunoassay" increased by using ATTO655 as a fluorescent dye and labeling the fluorescent dye to VH with a spacer interposed between them.

Furthermore, the present inventors conducted an experiment for determining a quenching effect by use of mutant VH in which four tryptophan residues (hereinafter sometimes referred to as Trp or W) present in VH are each substituted with phenylalanine residue (hereinafter sometimes referred to as Phe or F) and demonstrated that tryptophan residues at the positions 36, 47, 106 (corresponding to the position 103 in the Kabat numbering system) in an amino acid sequence of VH serve as a quencher for a fluorescent dye. Since these tryptophan residues are highly conserved in mouse antibody VH, it was found that an antigen could be measured by use of various antibodies if the "homogenous fluorescence based immunoassay" of the present invention is applied.

The present invention has been attained based on the aforementioned findings.

### Effect of the Invention

According to the present invention, it is possible to provide an immunoassay method enabling quick and simple quantitative measurement of a target substance in a liquid phase and a kit for measuring an antigen by the assay method. The assay method of the present invention is a method of detecting/measuring binding of an antigen, antibody VL and VH with reference to the fluorescence intensity of a fluorescent dye labeled to the antibody VL or VH and based on the novel finding that when the antibody VL and VH are not bound, the fluorescent dye is in a quenched state; whereas, when the antibody VL and VH are bound with an antigen interposed between them, the quenched state of the fluorescent dye is eliminated. This method requires neither a solid-phase immobilization step nor a washing step indispensable in conventional immunoassay methods. Thus, highly accurate measurement results with less variability can be obtained in a short time.

More specifically, the present invention relates to (1) an antigen-concentration measurement/detection kit having an antibody light-chain variable region polypeptide and an antibody heavy-chain variable region polypeptide, either one of which is labeled with a fluorescent dye, wherein the kit enables a measurement of antigen concentration or visualization of an antigen with reference to the positive correlation between the concentration of the antigen in a liquid phase and the fluorescence intensity of the fluorescent dye; and (2) the antigen-concentration measurement/detection kit according to (1) above, in which the antibody heavy-chain variable region polypeptide and the antibody light-chain variable region polypeptide are linked to form a single-chain antibody.

Furthermore, the present invention relates to (3) the antigen-concentration measurement/detection kit according to (1) or (2) above, in which the fluorescent dye is a rhodamine-based fluorescent dye or an oxazine-based fluorescent dye; (4) the antigen-concentration measurement/detection kit according to (3) above, in which the fluorescent dye is CR110, TAMRA or ATTO655; (5) the antigen-concentration measurement/detection kit according to any one of (1) to (4) above, in which the antibody heavy-chain variable region polypeptide includes a polypeptide consisting of the amino acid sequence represented by SEQ ID No:1 and the antibody light-chain variable region polypeptide includes a polypeptide consisting of the amino acid sequence represented by SEQ ID No:2; and (6) the antigen-concentration measurement/detection kit according to any one of (1) to (4) above, in which the antibody heavy-chain variable region polypeptide includes a polypeptide consisting of the amino acid sequence represented by SEQ ID No:6 and the antibody light-chain variable region polypeptide includes a polypeptide consisting of the amino acid sequence represented by SEQ ID No:7.

Furthermore, the present invention relates to (7) an antigen-concentration measurement/detection method comprising the following steps (a) to (c) sequentially in this order: (a) bringing an antibody light-chain variable region polypeptide and an antibody heavy-chain variable region polypeptide labeled with a fluorescent dye or an antibody heavy-chain variable region polypeptide and an antibody light-chain variable region polypeptide labeled with a fluorescent dye into contact with (a1) an antigen in a test substance in a liquid phase, or (a2) an antigen in a sample of a non-human animal test subject administered with the antibody light-chain variable region polypeptide and the antibody heavy-chain variable region polypeptide labeled with a fluorescent dye or the antibody heavy-chain variable region polypeptide and the antibody light-chain variable region polypeptide labeled with a fluorescent dye, or (a3) an antigen of a test subject *in vitro*; (b) measuring the fluorescence intensity of the fluorescent dye in the case of (a1) and detecting fluorescence of the fluorescent dye in the cases of (a2) and (a3); and (c) computationally obtaining the level of the antigen contained in the test substance in the case of (a1) and visualizing the antigen contained in the test subject in the cases of (a2) and (a3), with reference to the positive correlation between the concentration of the antigen in the liquid phase and the fluorescence intensity of the fluorescent dye.

Furthermore, the present invention relates to (8) the antigen-concentration measurement/detection method according to (7) above, in which the antibody heavy-chain variable region polypeptide and the antibody light-chain variable region polypeptide are linked to form a single-chain antibody; (9) the antigen-concentration measurement/detection method according to (7) or (8) above, in which the fluorescent dye is a rhodamine-based fluorescent dye or an oxazine-based fluorescent dye; (10) the antigen-concentration measurement/detection method according to (9) above, in which the fluorescent dye is CR110, TAMRA or ATTO655; (11) the antigen-concentration measurement/detection method according any one of (7) to (10) above, in which the antibody heavy-chain variable region polypeptide includes a polypeptide consisting of the amino acid sequence represented by SEQ ID No:1, and the antibody light-chain variable region polypeptide includes a polypeptide consisting of the amino acid sequence represented by SEQ ID No:2; and (12) the antigen-concentration measurement/detection method according any one of (7) to (10) above, in which the antibody heavy-chain variable region polypeptide includes a polypeptide consisting of the amino acid sequence represented by SEQ ID No:6 and the antibody light-chain variable region polypeptide includes a polypeptide consisting of the amino acid sequence represented by SEQ ID No:7.

### Brief Description of Drawings

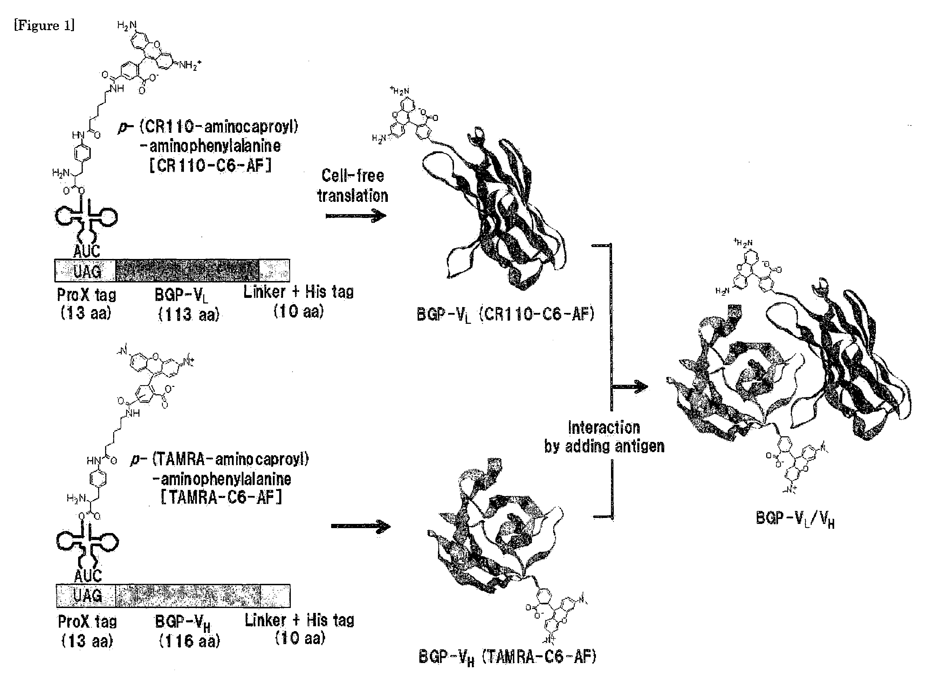

[Figure 1] This is a figure schematically showing CR110-labeled anti-BGP antibody light-chain variable region polypeptide (CR110-VL); TAMRA-labeled anti-BGP antibody heavy-chain variable region polypeptide (TAMRA-VH) and a complex thereof (CR110-VL/TAMRA-VH).

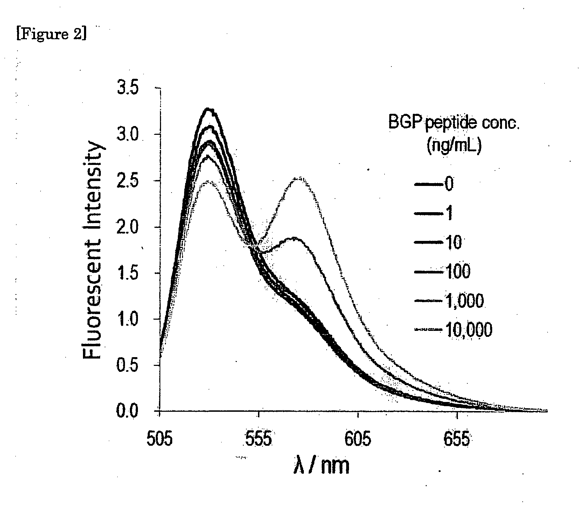

[Figure 2] CR110-VL and TAMRA-VH were allowed to react in the presence of BGP peptides in different concentrations and fluorescence spectra were measured at excitation light of 490 nm. The measurement results are shown in the figure.

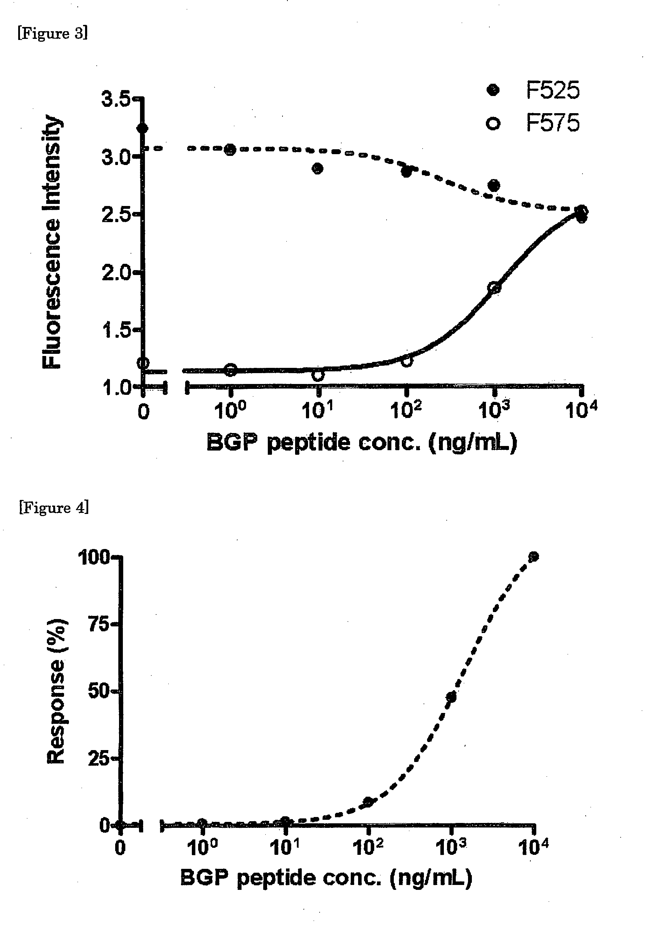

[Figure 3] CR110-VL and TAMRA-VH were allowed to react in the presence of BGP peptides in different concentrations and fluorescence intensity at 525 nm (F525) and 575 nm (F575) was measured. The resultant change in fluorescence intensity is shown in the figure.
[Figure 4] CR110-VL and TAMRA-VH were allowed to react in the presence of BGP peptides in different concentrations. The fluorescence intensity at 525 nm (F525) and 575 nm (F575) was measured and a change in fluorescence intensity ratio (F575/F525) was analyzed. The results are shown in the figure.

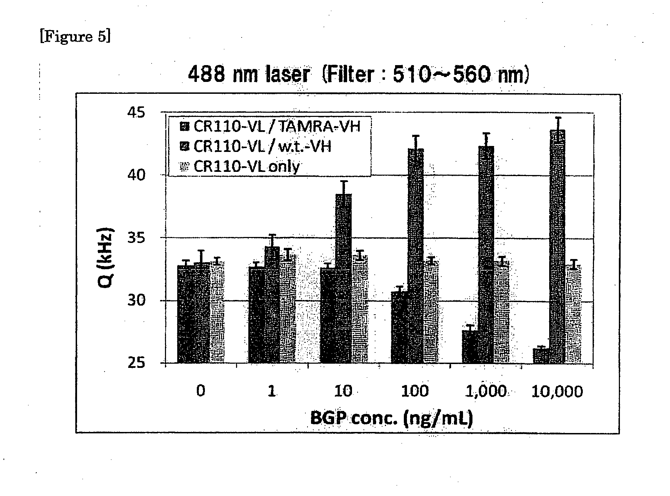

[Figure 5] A CR110-labeled anti-BGP antibody light-chain variable region polypeptide and a TAMRA-labeled anti-BGP antibody heavy-chain variable region polypeptide were allowed to react in the presence of BGP peptides in different concentrations. Analysis was made in accordance with fluorescence intensity multiple distribution analysis (FIDA) using a 488 nm laser and a 510 to 560 nm fluorescence filter. The results are shown in the figure. In the figure, CR110-VL represents the CR110-labeled anti-BGP antibody light-chain variable region polypeptide; TAMRA-VH represents the TAMRA-labeled anti-BGP antibody heavy-chain variable region polypeptide; and w.t.-VH represents a non-labeled anti-BGP antibody heavy-chain variable region polypeptide.

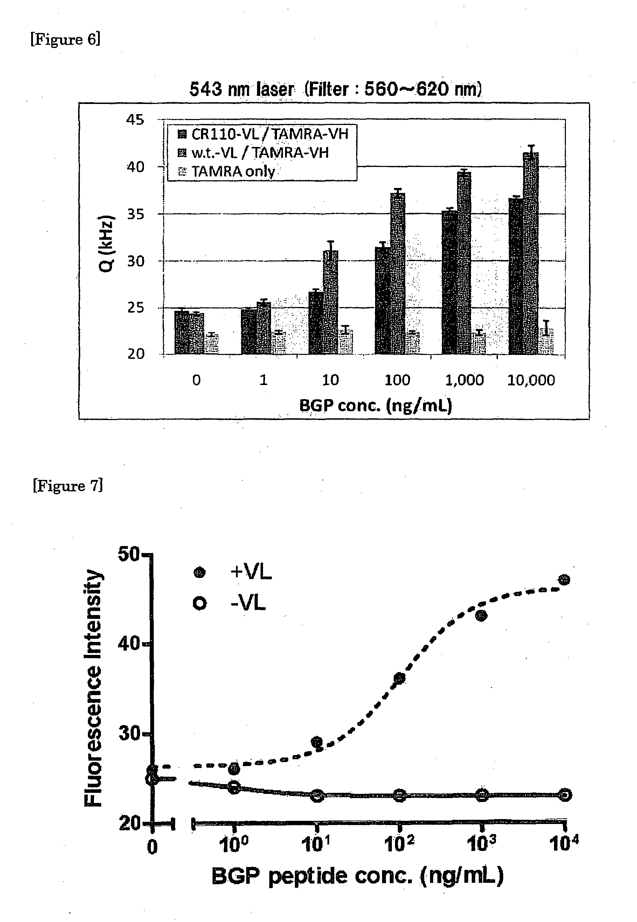

[Figure 6] A CR110-labeled anti-BGP antibody light-chain variable region polypeptide and a TAMRA-labeled anti-BGP antibody heavy-chain variable region polypeptide were allowed to react in the presence of BGP peptides in different concentrations and analysis was made in accordance with the fluorescence intensity multiple distribution analysis (FIDA) using a 543 nm laser and a 560 to 620 nm fluorescence filter. The results are shown in the figure. In the figure, CR110-VL represents the CR110-labeled anti-BGP antibody light-chain variable region polypeptide; TAMRA-VH represents the TAMRA-labeled anti-BGP antibody heavy-chain variable region polypeptide; and w.t.-VL represents a non-labeled anti-BGP antibody light-chain variable region polypeptide.
[Figure 7] TAMRA-labeled anti-BGP antibody heavy-chain variable region polypeptide was allowed to react with BGP peptides in different concentrations in the presence or absence of non-labeled anti-BGP antibody light-chain variable region polypeptide and fluorescence intensity was measured by use of 543 nm He-Ne laser. The results are shown in the figure. In the figure, +VL represents the results in the case where a reaction was performed in the presence of a non-labeled anti-BGP antibody light-chain variable region polypeptide; -VL represents the measurement results of fluorescence intensity in the case where a reaction was performed in the absence of non-labeled anti-BGP antibody light-chain variable region polypeptide.

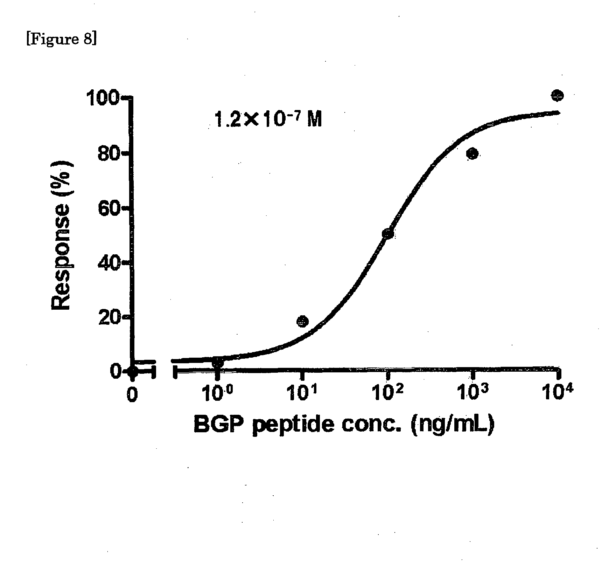

[Figure 8] A TAMRA-labeled anti-BGP antibody heavy-chain variable region polypeptide and BGP peptides in different concentrations were allowed to react in the presence or absence of a non-labeled anti-BGP antibody light-chain variable region polypeptide and the fluorescence-intensity ratio (+VL/-VL) was analyzed. The results are shown in the figure.

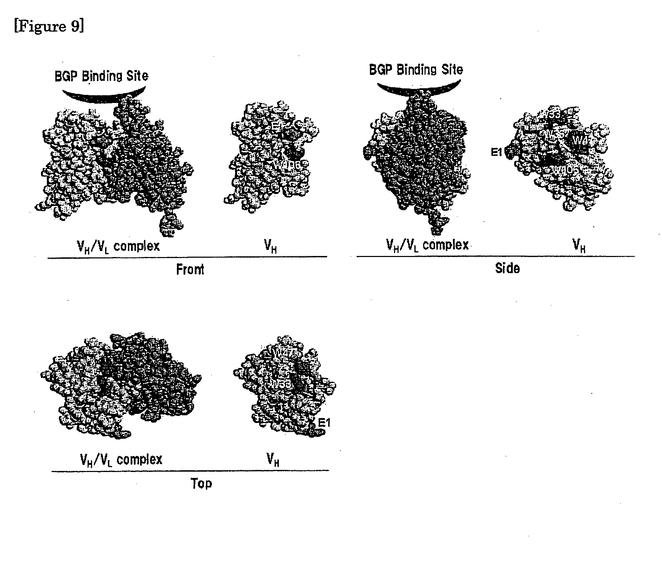

[Figure 9] Tryptophan residues present in an anti-BGP antibody heavy-chain variable region polypeptide are shown in the figure. The figure shows the positions of tryptophan residues (W33, W36, W47, W106) in three-dimensional structure prediction models of a complex (VH/VL complex) of an anti-BGP antibody heavy-chain variable region polypeptide and an anti-BGP antibody light-chain variable region polypeptide, and a single anti-BGP antibody heavy-chain variable region polypeptide (VH). Note that the positions of these tryptophan residues respectively correspond to the amino acid positions 33, 36, 47, 103 in VH according to the Kabat numbering system.

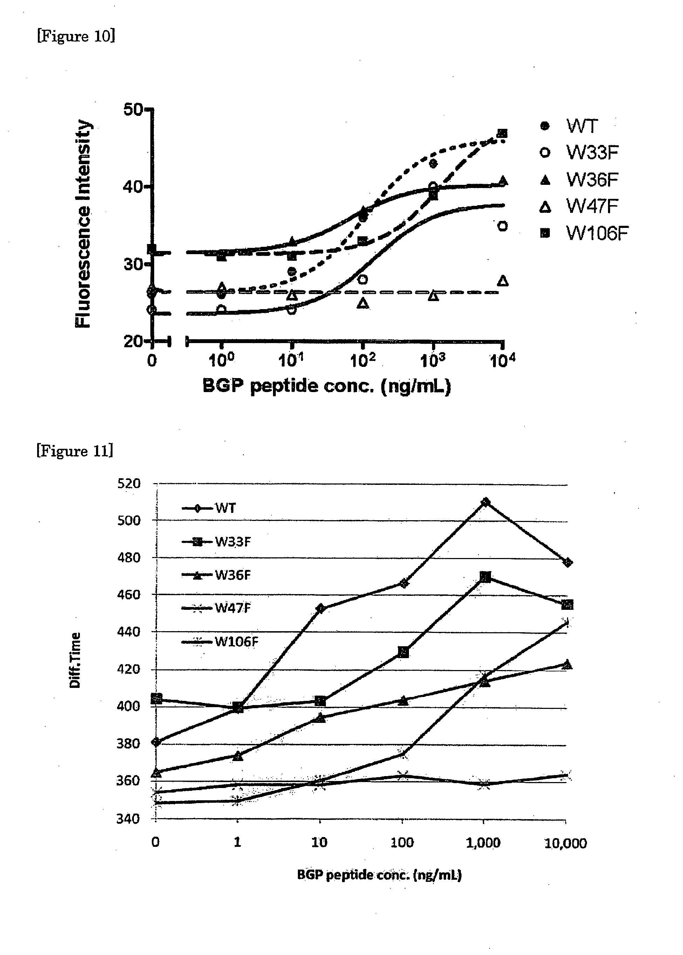

[Figure 10] Either the wild-type (WT) or a mutant anti-BGP antibody heavy-chain variable region polypeptide (W33F, W36F, W47F, or W106F), and an anti-BGP antibody light-chain variable region polypeptide were allowed to react in the presence of BGP peptide in different concentration, and fluorescence intensity was measured by using a 543 nm He-Ne laser. The results are shown in the figure.
[Figure 11] Either the wild-type (WT) or a mutant anti-BGP antibody heavy-chain variable region polypeptide (W33F, W36F, W47F, or W106F) and an anti-BGP antibody light-chain variable region polypeptide were allowed to react in the presence of BGP peptides in different concentrations, and a change in diffusion time was analyzed by a Fluorescence Correlation Spectroscopy (FCS). The results are shown in the figure.

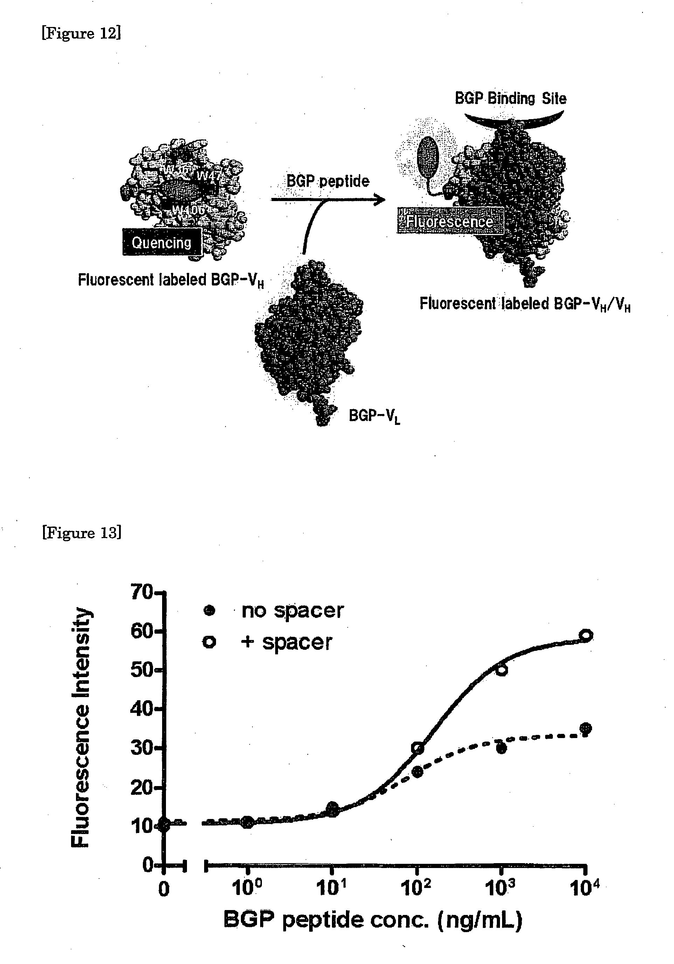

[Figure 12] This is a figure showing a three-dimensional structural prediction model of the complex (Fluorescent-labeled BGP-VH/VL) of a spacer-added fluorescent-labeled anti-BGP antibody heavy-chain variable region polypeptide (Fluorescent-labeled BGP-VH) and an anti-BGP antibody light-chain variable region polypeptide (BGP-VL).
[Figure 13] ATT0655-labeled anti-BGP antibody heavy-chain variable region polypeptide containing or not containing a spacer (GGGSGGGS; SEQ ID No:4) and an anti-BGP antibody light-chain variable region polypeptide were allowed to react in the presence of BGP peptides in different concentrations, and fluorescence intensity was measured. The results are shown in the figure.
[Figure 14] ATT0655-labeled anti-BGP antibody heavy-chain variable region polypeptide containing or not containing a spacer (GGGSGGGS; SEQ ID No:4) and an anti-BGP antibody light-chain variable region polypeptide were allowed to react in the presence of BGP peptides in different concentrations. The ratio of fluorescence intensity measured is shown in the figure.

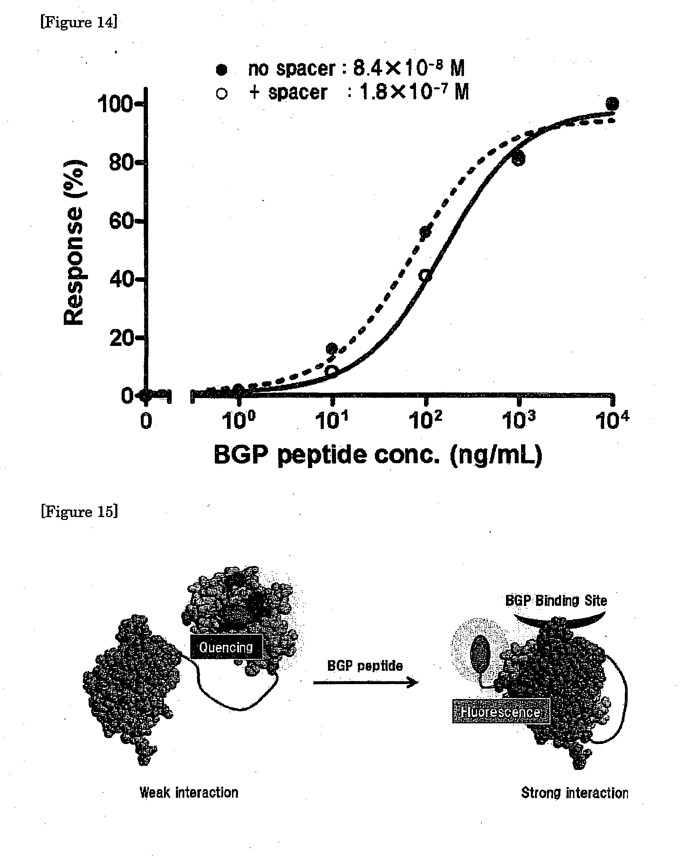

[Figure 15] This is a figure showing a three-dimensional structural prediction model of a fluorescent-labeled single-chain antibody in which a fluorescent-labeled antibody heavy-chain variable region polypeptide and an antibody light-chain variable region polypeptide are linked.

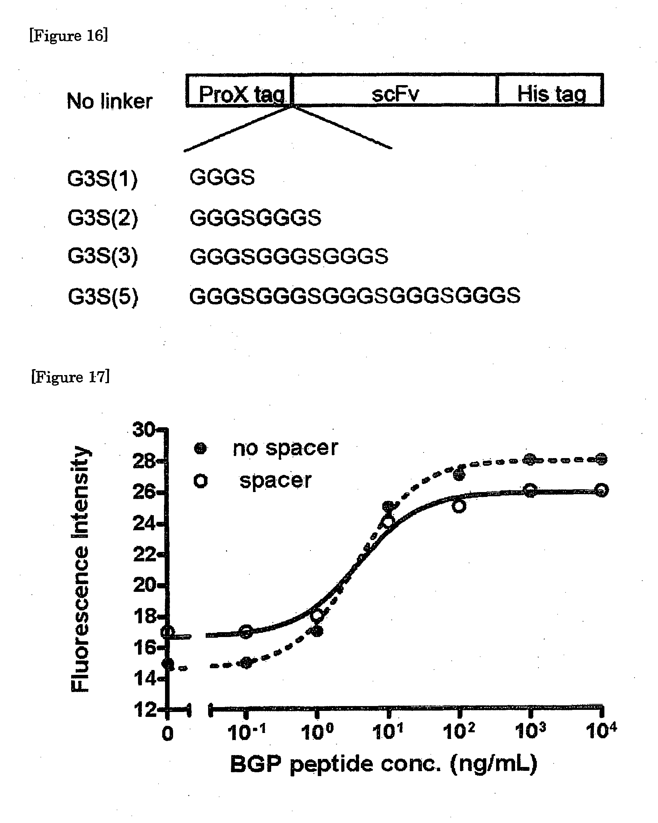

[Figure 16] This is a schematic figure of a primary structure of a fluorescent-labeled single-chain antibody in which a fluorescent-labeled antibody heavy-chain variable region polypeptide and an antibody light-chain variable region polypeptide are linked.
[Figure 17] An ATT0655-labeled anti-BGP single-chain antibody containing or not containing a spacer and BGP peptides in different concentrations were allowed to react. The results of measured fluorescence intensity are shown in the figure.

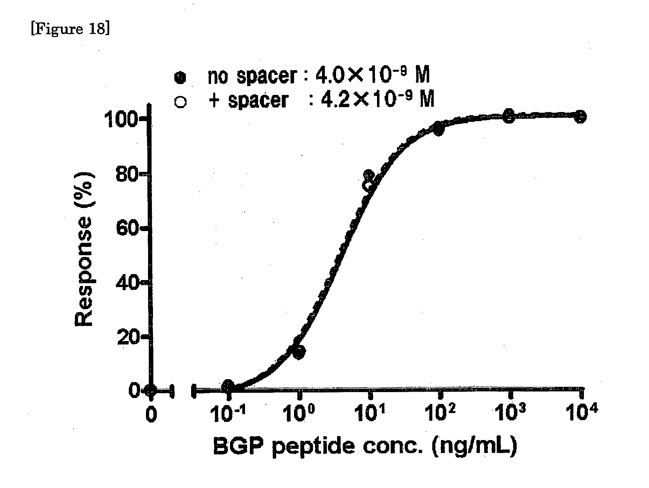

[Figure 18] An ATTO655-labeled anti-BGP single-chain antibody containing or not containing a spacer and BGP peptides in different concentrations were allowed to react. The ratio of fluorescence intensity measured is shown in the figure.
[Figure 19] An ATT0655-labeled anti-BGP single-chain antibody and BGP peptides in different concentrations were allowed to react, and fluorescence was detected by a fluorescence image analyzer (FMBIO-III; manufactured by Hitachi Software Engineering). The results are shown in the figure. In the figure, FL92 represents a spacer sequence consisting of the amino acid sequence represented by SEQ ID No:3; and 2TAG represents a spacer sequence consisting of MX (X represents a fluorescent-labeled amino acid).

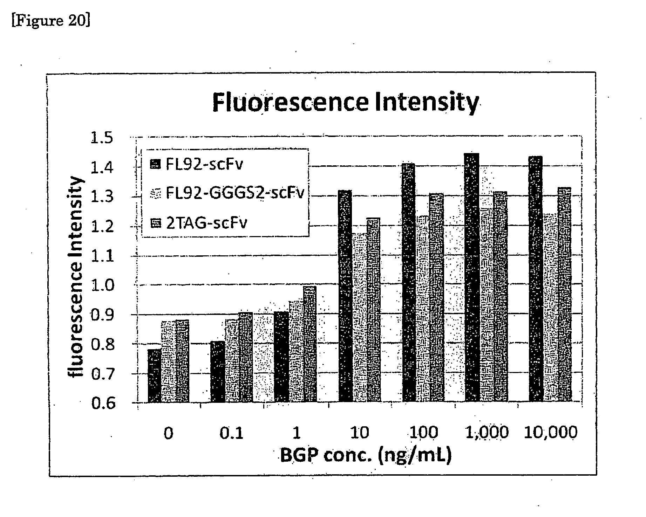

[Figure 20] A fluorescent-labeled anti-BGP single-chain antibody containing or not containing a spacer, and BGP peptides in different concentrations were allowed to react, and fluorescence was quantitatively determined by the fluorescence image analyzer (FMBIO-III; manufactured by Hitachi Software Engineering). The results are shown in the figure. In the figure, FL92 represents a spacer sequence consisting of the amino acid sequence represented by SEQ ID No:3; and 2TAG represents a spacer sequence consisting of MX (X represents a fluorescent-labeled amino acid).

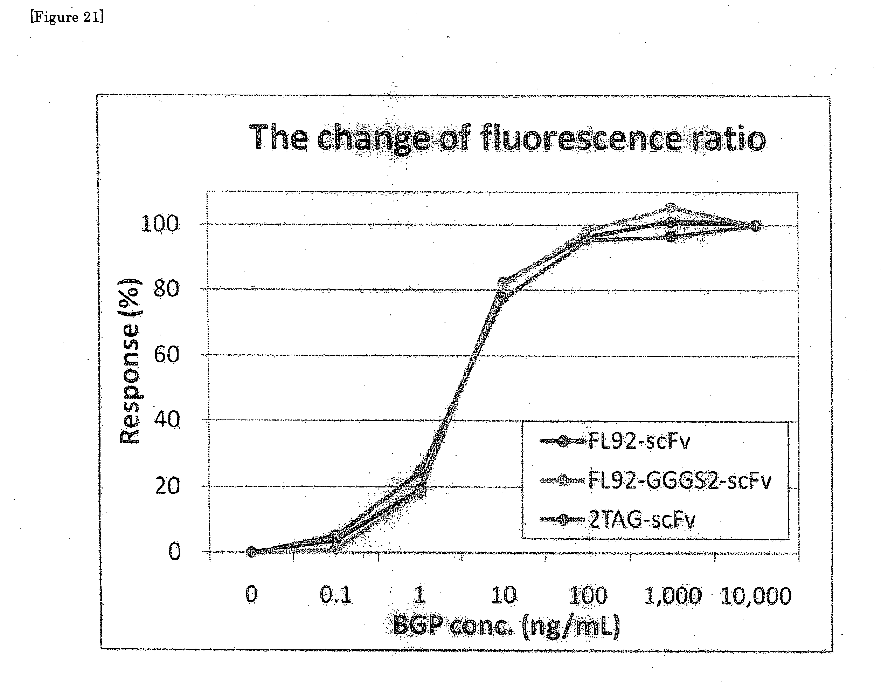

[Figure 21] A fluorescent-labeled anti-BGP single-chain antibody containing or not containing a spacer, and BGP peptides in different concentrations were allowed to react, and fluorescence was detected by the fluorescence image analyzer (FMBIO-III; manufactured by Hitachi Software Engineering). The results are shown in the figure. In the figure, FL92 represents a spacer sequence consisting of the amino acid sequence represented by SEQ ID No:3; and 2TAG represents a spacer sequence consisting of MX (X represents a fluorescent-labeled amino acid).

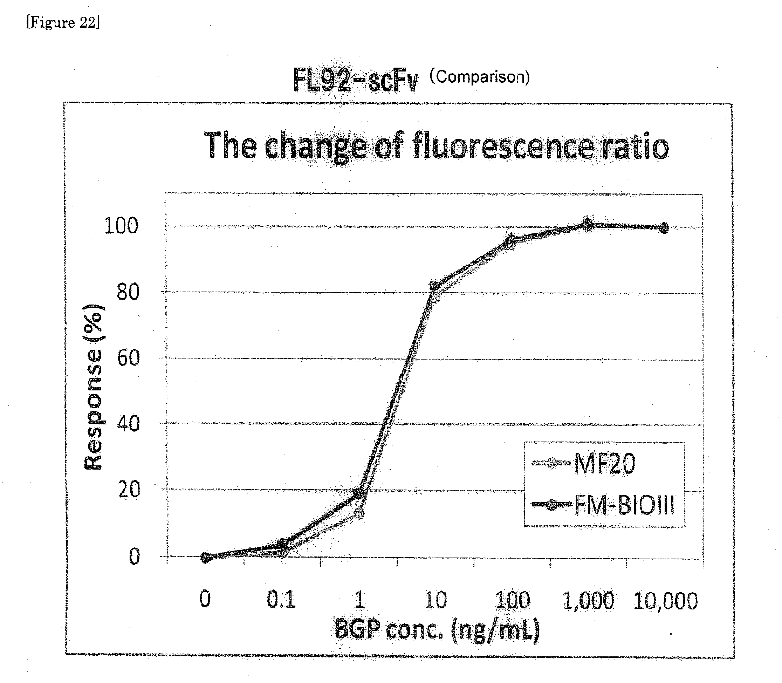

[Figure 22] A fluorescent-labeled anti-BGP single-chain antibody containing FL92 spacer (SEQ ID No:3), and BGP peptides in different concentrations were allowed to react. The ratio of fluorescence intensity measured by the fluorescence image analyzer (FMBIO-III; manufactured by Hitachi Software Engineering) and MF20/FluoroPoint-Light (manufactured by Olympus Corporation) is shown in the figure.

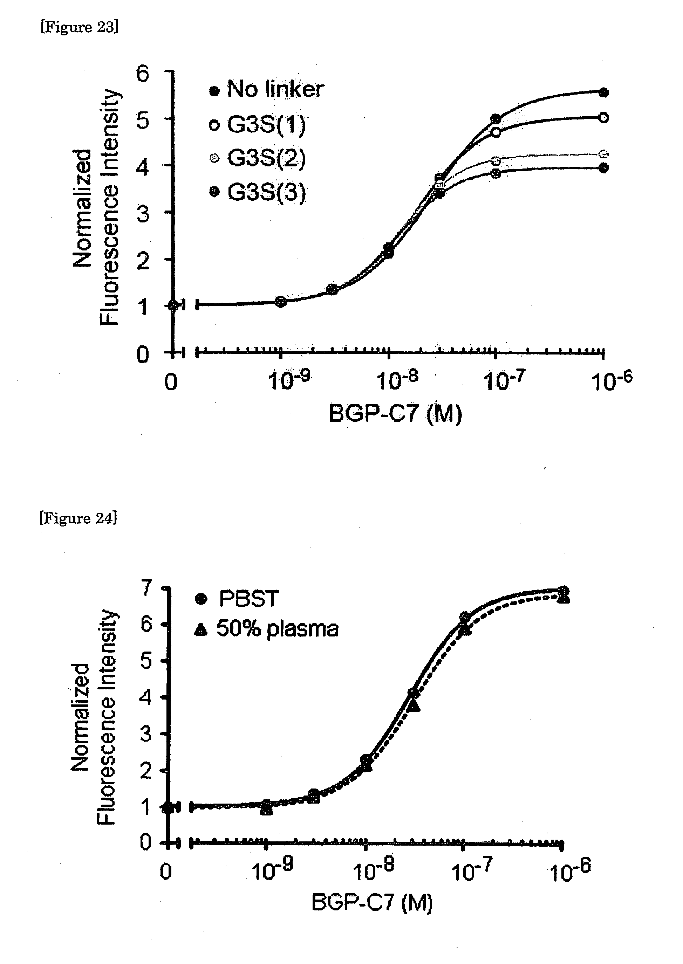

[Figure 23] A TAMRA-labeled anti-BGP single-chain antibody containing or not containing a spacer, and BGP peptides in different concentrations were allowed to react, and fluorescence was detected. The results are shown in the figure. G3S (1) indicates that G3S contains a spacer (linker) having a sequence represented by GGGS; G3S (2) indicates that G3S contains a spacer (linker) having a sequence represented by GGGSGGGS (SEQ ID No:4); and G3S (3) indicates that G3S contains a spacer (linker) having a sequence represented by GGGSGGGSGGGS (SEQ ID No:10).
[Figure 24] A TAMRA-labeled anti-BGP single-chain antibody protein and BGP in different concentrations were reacted in the presence of a PBST buffer solution and the human plasma of a final concentration of 50%, and fluorescence intensity was measured. The results are shown in the figure.
[Figure 25] An ATT0655-labeled anti-bisphenol A (BPA) antibody heavy-chain variable region polypeptide and BPA in different concentrations were allowed to react in the presence or absence of a non-labeled anti-BPA antibody light-chain variable region polypeptide and fluorescence intensity was measured. The results are shown in the figure.
[Figure 26] An ATT0655-labeled anti-BPA antibody heavy-chain variable region polypeptide and BPA in different concentrations were allowed to react in the presence or absence of a non-labeled anti-BPA antibody light-chain variable region polypeptide, and fluorescence intensity was measured. The ratio of the fluorescence intensity measured is shown in the figure.

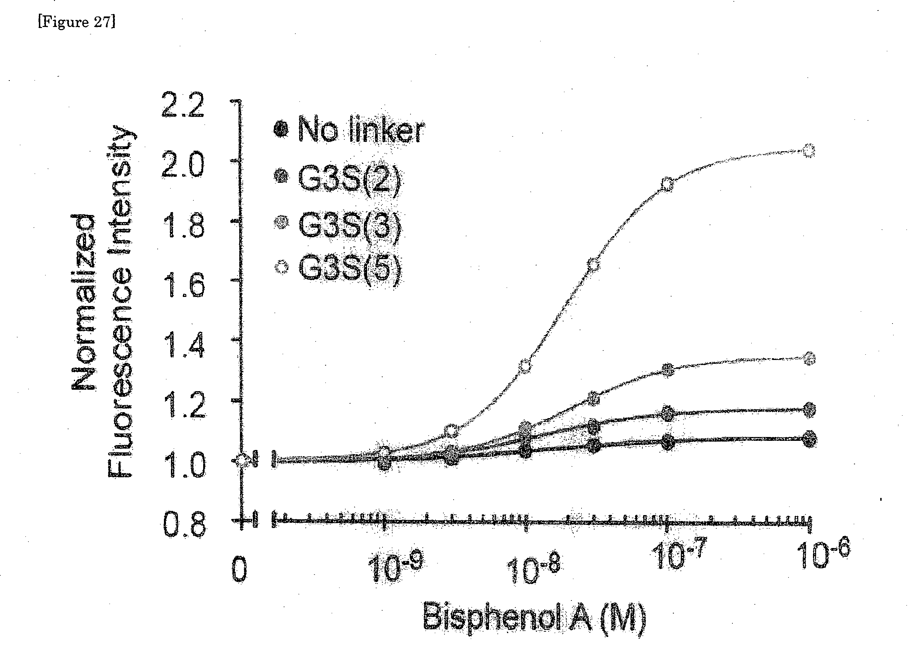

[Figure 27] A TAMRA-labeled anti-BPA single-chain antibody containing or not containing a spacer and BPA peptides in different concentrations were allowed to react, and fluorescence was detected. The results are shown in the figure. G3S (2) indicates that G3S contains a spacer (linker) having a sequence represented by GGGSGGGS (SEQ ID No:4); G3S (3) indicates that G3S contains a spacer (linker) having a sequence represented by GGGSGGGSGGGS (SEQ ID No:10); and G3S (5) indicates that G3S contains a spacer (linker) having a sequence represented by GGGSGGGSGGGSGGGSGGGS (SEQ ID No:11).

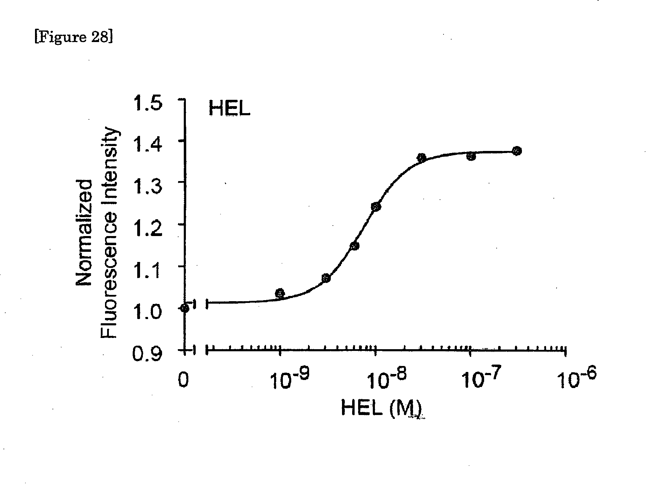

[Figure 28] A TAMRA-labeled anti-HEL single-chain antibody and HEL proteins in different concentrations were allowed to react and fluorescence was detected. The results are shown in the figure.

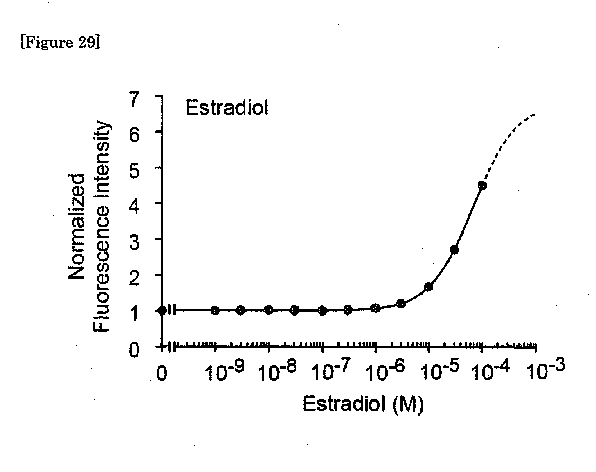

[Figure 29] A TAMRA-labeled anti-estradiol single-chain antibody and estradiol in different concentrations were allowed to react and fluorescence was detected. The results are shown in the figure.

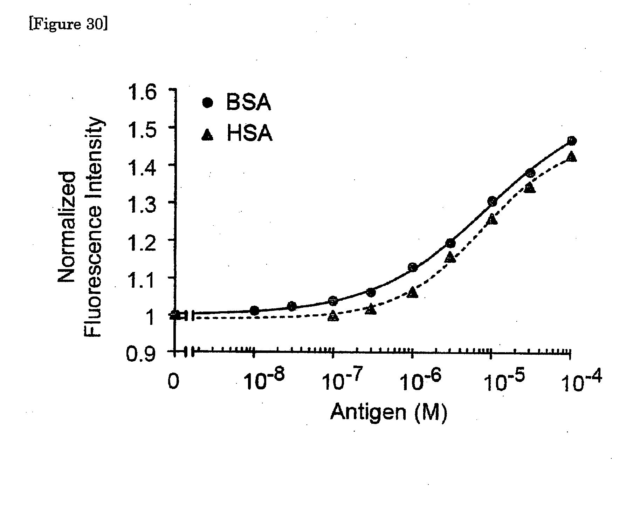

[Figure 30] A TAMRA-labeled anti-SA single-chain antibody and BSA or HSA in different concentrations were allowed to react and fluorescence was detected. The results are shown in the figure.

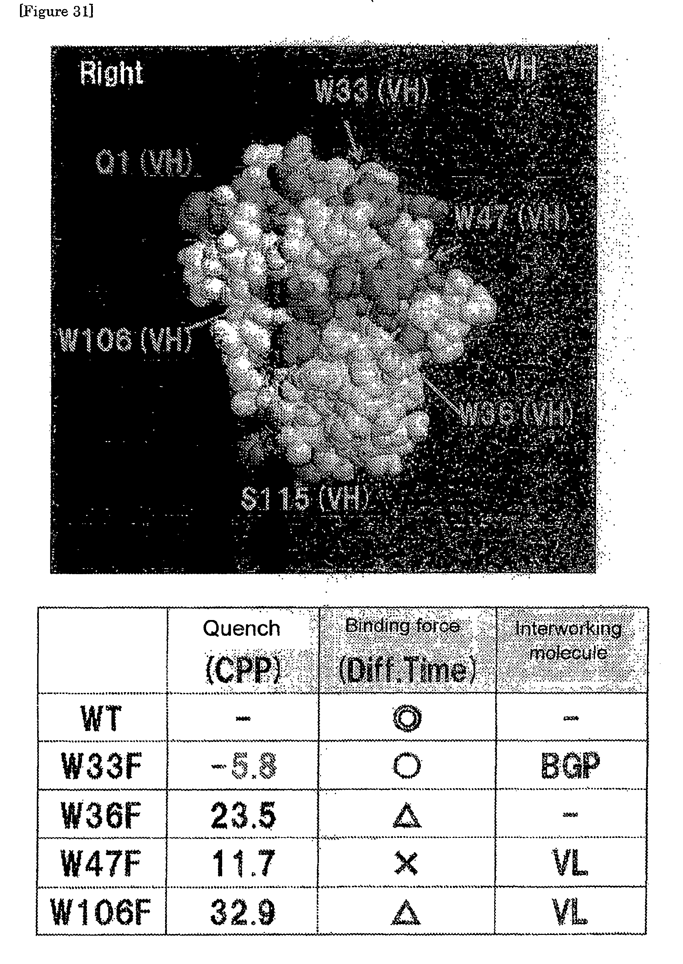

[Figure 31] This is a figure showing that tryptophane at the position 36, 47 or 103 according to the Kabat numbering system is conserved in the amino acid sequence of a mouse antibody heavy-chain variable region. The figure shows a three-dimensional structural prediction model of an anti-BGP mouse antibody heavy-chain variable region and the possibility that W36, W47, W106 of the tryptophan residues contained in the anti-BGP mouse antibody heavy-chain variable region are particularly involved in the quenching.

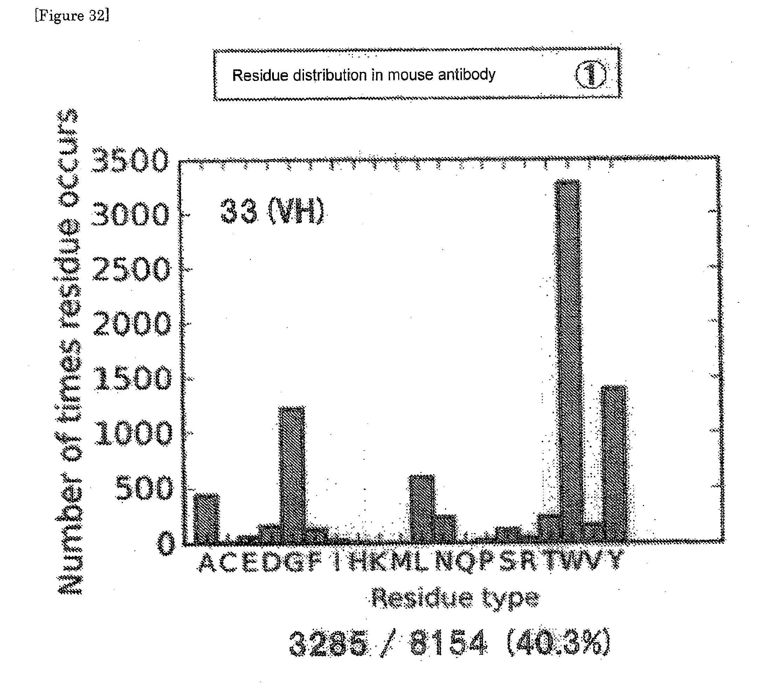

[Figure 32] This is a figure showing that W33 is highly conserved in many types of mouse antibody heavy-chain variable regions.

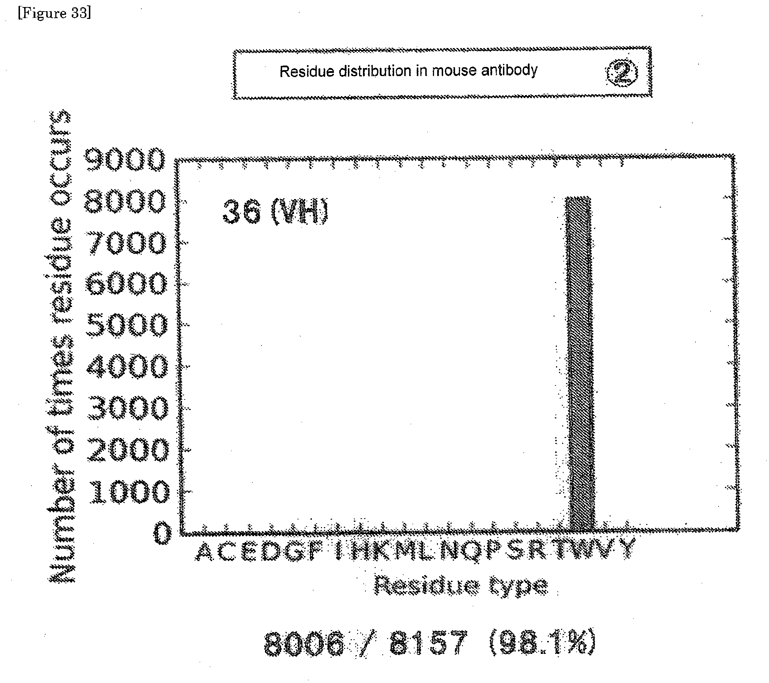

[Figure 33] This is a figure showing that W36 is highly conserved in many types of mouse antibody heavy-chain variable regions.

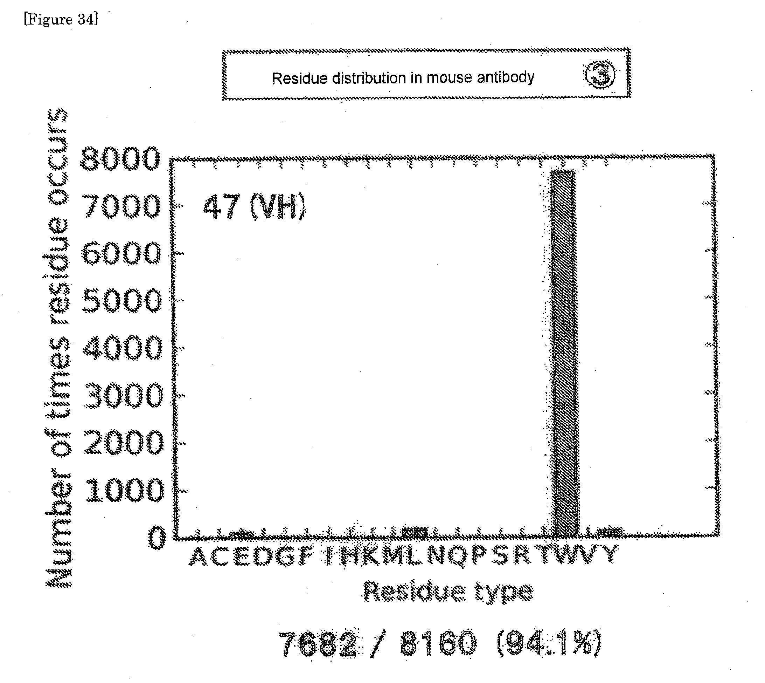

[Figure 34] This is a figure showing that W47 is highly conserved in many types of mouse antibody heavy-chain variable regions.

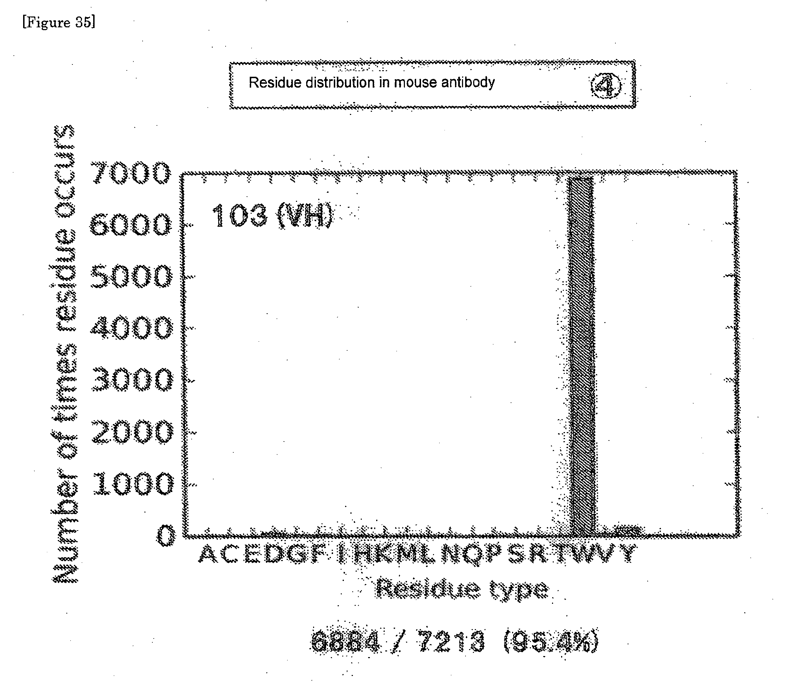

[Figure 35] This is a figure showing that W106 is highly conserved in many types of mouse antibody heavy-chain variable regions. Note that Trp106 in the amino acid sequence of the above VH corresponds to the 103rd position according to the Kabat numbering system.

### Mode for Carrying Out the Invention

An antigen-concentration measurement/detection kit of the present invention is a kit having an antibody light-chain variable region polypeptide and an antibody heavy-chain variable region polypeptide, either one of which is labeled with a fluorescent dye, wherein the kit enables a measurement of antigen concentration or visualization of an antigen with reference to the positive correlation between the concentration of the antigen in a liquid phase and the fluorescence intensity of the fluorescent dye as claimed.
More specifically, the antigen-concentration measurement/detection kit belongs to the following kits:
(1) an antigen concentration measurement kit having an antibody light-chain variable region polypeptide and an antibody heavy-chain variable region polypeptide labeled with a fluorescent dye, wherein the kit enables a measurement of the concentration of an antigen with reference to the positive correlation between the concentration of the antigen in a liquid phase and the fluorescence intensity of the fluorescent dye;
(2) an antigen detection kit having an antibody light-chain variable region polypeptide and an antibody heavy-chain variable region polypeptide labeled with a fluorescent dye, wherein the kit enables a visualization of an antigen with reference to the positive correlation between the level of the antigen in a test subject and the fluorescence intensity of the fluorescent dye;
(3) an antigen concentration measurement kit having an antibody heavy-chain variable region polypeptide and an antibody light-chain variable region polypeptide labeled with a fluorescent dye, wherein the kit enables a measurement of the concentration of an antigen with reference to the positive correlation between the concentration of the antigen in a liquid phase and the fluorescence intensity of the fluorescent dye; and
(4) a kit having an antibody heavy-chain variable region polypeptide and an antibody light-chain variable region polypeptide labeled with a fluorescent dye, wherein the kit enables a visualization of an antigen with reference to the positive correlation between the level of the antigen in a test subject and the fluorescence intensity of the fluorescent dye.

The antibody light-chain variable region polypeptide and the antibody heavy-chain variable region polypeptide may be prepared as two mutually independent polypeptide fragments as long as they can form a complex with a same antigen molecule interposed between them or may be prepared as a fused single-chain antibody with a linker, etc. interposed between them. Furthermore, the antigen is not particularly limited as long as it can be specifically recognized by the antibody heavy-chain variable region polypeptide and the antibody light-chain variable region polypeptide. Examples of the antigen include a protein, a peptide, a saccharide, a lipid, a glycolipid and a low molecular compound.

An antigen-concentration measurement/detection method of the present invention is an antigen-concentration measurement/detection method comprising the following steps (a) to (c) sequentially in this order: (a) bringing an antibody light-chain variable region polypeptide and an antibody heavy-chain variable region polypeptide labeled with a fluorescent dye or an antibody heavy-chain variable region polypeptide and an antibody light-chain variable region polypeptide labeled with a fluorescent dye into contact with (a1) an antigen in a test substance in a liquid phase, or (a2) an antigen in a sample of a non-human animal test subject administered with the antibody light-chain variable region polypeptide and the antibody heavy-chain variable region polypeptide labeled with a fluorescent dye or the antibody heavy-chain variable region polypeptide and the antibody light-chain variable region polypeptide labeled with a fluorescent dye, or in (a3) an antigen of a test subject *in vitro*; (b) measuring the fluorescence intensity of the fluorescent dye in the case of (a1) and detecting fluorescence of the fluorescent dye in the cases of (a2) and (a3); and (c) computationally obtaining the level of the antigen contained in the test substance in the case of (a1) and visualizing the antigen contained in the test subject in the cases of (a2) and (a3), with reference to the positive correlation between the concentration of the antigen in a liquid phase and the fluorescence intensity of the fluorescent dye.

More specifically, the antigen-concentration measurement/detection method belongs to the following methods:
an antigen-concentration measuring method (hereinafter sometimes referred to as "measurement method [I]"), comprising the following steps, sequentially in this order:
   (a1-1) bringing an antibody light-chain variable region polypeptide and an antibody heavy-chain variable region polypeptide labeled with a fluorescent dye into contact with an antigen in a test substance, in a liquid phase,
   (b) measuring the fluorescence intensity of the fluorescent dye, and
   (c) computationally obtaining the level of the antigen contained in the test substance; that is, a step of computationally obtaining the level of the antigen contained in a test substance with reference to the positive correlation between the concentration of the antigen in a liquid phase and the fluorescence intensity of the fluorescent dye;
an antigen-concentration measuring method (hereinafter sometimes referred to as "measurement method [II]") comprising the following steps, sequentially in this order:
   (a1-2) bringing an antibody heavy-chain variable region polypeptide and an antibody light-chain variable region polypeptide labeled with a fluorescent dye into contact with an antigen in a test substance, in a liquid phase,
   (b) measuring the fluorescence intensity of the fluorescent dye, and
   (c) computationally obtaining the level of the antigen contained in the test substance; that is, a step of computationally obtaining the level of the antigen contained in a test substance with reference to the positive correlation between the concentration of the antigen in a liquid phase and the fluorescence intensity of the fluorescent dye;
an antigen detection method (hereinafter sometimes referred to as "non-human animal detection method [I]") comprising the following steps, sequentially in this order:
   (a2-1) bringing an antibody light-chain variable region polypeptide and an antibody heavy-chain variable region polypeptide labeled with a fluorescent dye into contact with an antigen in a non-human animal test subject administered with the antibody light-chain variable region polypeptide and the antibody heavy-chain variable region polypeptide labeled with a fluorescent dye,
   (b) detecting the fluorescence of the fluorescent dye, and
   (c) visualizing the antigen contained in the test subject, with reference to the positive correlation between the level of the antigen in the non-human animal test subject and the fluorescence intensity of the fluorescent dye;
an antigen detection method (hereinafter sometimes referred to as "non-human animal detection method [II]") comprising the following steps, sequentially in this order:
   (a2-2) bringing an antibody heavy-chain variable region polypeptide and an antibody light-chain variable region polypeptide labeled with a fluorescent dye into contact with an antigen in a non-human animal test subject administered with the antibody heavy-chain variable region polypeptide and the antibody light-chain variable region polypeptide labeled with a fluorescent dye,
   (b) detecting the fluorescence of the fluorescent dye, and
   (c) visualizing the antigen contained in the test subject, with reference to the positive correlation between the level of the antigen in the non-human animal test subject and the fluorescence intensity of the fluorescent dye;
an antigen detection method (hereinafter sometimes referred to as *"in vitro* detection method [I]") comprising the following steps, sequentially in this order:
   (a3-1) bringing an antibody light-chain variable region polypeptide and an antibody heavy-chain variable region polypeptide labeled with a fluorescent dye into contact with an antigen in a test subject *in vitro;*
   (b) detecting the fluorescence of the fluorescent dye, and
   (c) visualizing the antigen contained in the test subject, with reference to the positive correlation between the level of the antigen in the test subject and the fluorescence intensity of the fluorescent dye; and
an antigen detection method (hereinafter sometimes referred to as *"in vitro* detection method [II]") comprising the following steps, sequentially in this order:
   (a3-2) bringing an antibody heavy-chain variable region polypeptide and an antibody light-chain variable region polypeptide labeled with a fluorescent dye into contact with an antigen in a test subject *in vitro;*
   (b) detecting the fluorescence of the fluorescent dye, and
   (c) visualizing the antigen contained in the test subject, with reference to the positive correlation between the level of the antigen in the test subject and the fluorescence intensity of the fluorescent dye.

The antibody light-chain variable region polypeptide and the antibody heavy-chain variable region polypeptide may be prepared as mutually independent two polypeptide fragments as long as they can form a complex with a same antigen molecule interposed between them or may be prepared as a fused single-chain antibody with a linker, etc. interposed between them. Furthermore, the antigen is not particularly limited as long as it can be specifically recognized by the antibody heavy-chain variable region polypeptide and the antibody light-chain variable region polypeptide. Examples of the antigen include a protein, a peptide, a saccharide, a lipid, a glycolipid and a low molecular compound.

The antibody heavy-chain variable region polypeptide is not particularly limited as long as it contains an amino acid sequence specific to an antibody heavy-chain variable region encoded by exons of the V-region, D region and J region of an antibody heavy chain gene. An arbitral amino acid sequence may be further added to an N-terminal and/or C-terminal side of the amino acid sequence specific to the antibody heavy-chain variable region. Furthermore, as the amino acid sequence specific to the antibody heavy-chain variable region, an amino acid sequence having tryptophan as the amino acid positions 36, 47, 103 according to the Kabat numbering system is preferred. More specifically, the amino acid sequence represented by SEQ ID No:1 and the amino acid sequence represented by SEQ ID No:6 are preferably exemplified.

The antibody light-chain variable region polypeptide is not particularly limited as long as it contains an amino acid specific to an antibody light-chain variable region encoded by exons of V-region and J region of the antibody light-chain gene. An arbitral amino acid sequence may be further added to the N-terminal and/or C-terminal side of the amino acid sequence specific to the antibody light-chain variable region. Furthermore, as the amino acid sequence specific to the antibody light-chain variable region, an amino acid sequence having tryptophan as the 35th amino acid according to the Kabat numbering system is preferred. More specifically, the amino acid sequence represented by SEQ ID No:2 and the amino acid sequence represented by SEQ ID No:7 are preferably exemplified.

An antibody heavy-chain variable region polypeptide, an antibody light-chain variable region polypeptide and a single-chain antibody polypeptide containing both of an antibody heavy-chain variable region and an antibody light-chain variable region can be prepared by use of a known method such as a chemical synthesis method, a genetic recombination technique and a proteolytic decomposition method of an antibody molecule. Of them, the genetic recombination technique capable of preparing a polypeptide in a large amount and in a relatively easy operation is preferably employed for the preparation. When the polypeptide is prepared by the genetic recombination technique, DNA containing a nucleotide sequence encoding an amino acid sequence specific to an antibody light-chain variable region or an antibody light-chain variable region is introduced into a suitable vector to prepare an expression vector. By using an expression system of a host such as bacteria, yeast, an insect, an animal or plant cell and a cell-free translation system, a desired polypeptide can be expressed. When a polypeptide is expressed by the cell-free translation system, the polypeptide can be expressed in a reaction solution prepared by adding nucleotide triphosphates and various types of amino acids to a cell-free extract from e.g., *Escherichia coli,* wheat germ and rabbit reticulocyte.

The fluorescent dye is not particularly limited as long as it can be quenched in the state where it is labeled to an antibody heavy-chain variable region polypeptide or an antibody light-chain variable region polypeptide. Examples of the fluorescent dye include fluorescent dyes having rhodamine, coumarin, Cy, EvoBlue, oxazine, Carbopyronin, naphthalene, biphenyl, anthracene, phenenthrene, pyrene, carbazole, etc. as a basic skeleton and derivatives of these fluorescent dyes. Specific examples thereof include CR110: carboxyrhodamine 110: Rhodamine Green (trade name), TAMRA: carbocytetremethlrhodamine: TMR, ATTO655 (trade name), BODIPY FL (trade name): 4,4-difluoro-5,7-dimethyl-4-bora-3a, 4a-diaza-s-indancene-3-propionic acid, BODIPY 493/503 (trade name): 4,4-difluoro-1,3,5,7-tetramethyl-4-bora-3a,4a-diaza-s-indancene-8-propionicacid, BODIPY R6G (trade name): 4,4-difluoro-5-(4-phenyl-1,3-butadienyl)-4-bora-3a,4a-diaza-s-indancene-3-propionic acid, BODIPY 558/568 (trade name): 4,4-difluoro-5- (2-thienyl)-4-bora-3a,4a-diaza-s-indancene-3-propionic acid, BODIPY 564/570 (trade name): 4,4-difluoro-5-styryl-4-bora-3a,4a-diaza-s-indancene-3-propionic acid, BODIPY 576/589 (trade name): 4,4-difluoro-5-(2-pyrrolyl)-4-bora-3a,4a-diaza-s-indancene-3-propionic acid, BODIPY 581/591 (trade name) :4,4-difluoro-5-(4-phenyl-1,3-butadienyl)-4-bora-3a,4a-diaza-s-indancene-3-propionic acid, Cy3 (trade name), Cy3B (trade name), Cy3.5 (trade name), Cy5 (trade name), Cy5.5 (trade name), EvoBlue10 (trade name), EvoBlue30 (trade name), MR121, ATTO 390 (trade name), ATTO 425 (trade name), ATTO 465 (trade name), ATTO 488 (trade name), ATTO 495 (trade name), ATTO 520 (trade name), ATTO 532 (trade name), ATTO Rho6G (trade name), ATTO 550 (trade name), ATTO 565 (trade name), ATTO Rho3B (trade name), ATTO Rho11 (trade name), ATTO Rho12 (trade name), ATTO Thio12 (trade name), ATTO 610 (trade name), ATTO 611X (trade name), ATTO 620 (trade name), ATTO Rho14 (trade name), ATTO 633 (trade name), ATTO 647 (trade name), ATTO 647N (trade name), ATTO 655 (trade name), ATTO Oxa12 (trade name), ATTO 700 (trade name), ATTO 725 (trade name), ATTO 740 (trade name), Alexa Fluor 350 (trade name), Alexa Fluor 405 (trade name), Alexa Fluor 430 (trade name), Alexa Fluor 488 (trade name), Alexa Fluor 532 (trade name), Alexa Fluor 546 (trade name), Alexa Fluor 555 (trade name), Alexa Fluor 568 (trade name), Alexa Fluor 594 (trade name), Alexa Fluor 633 (trade name), Alexa Fluor 647 (trade name), Alexa Fluor 680 (trade name), Alexa Fluor 700 (trade name), Alexa Fluor 750 (trade name), Alexa Fluor 790 (trade name), Rhodamine Red-X (trade name), Texas Red-X (trade name), 5 (6)-TAMRA-X (trade name), 5TAMRA (trade name) and SFX (trade name). Of them, a rhodamine-based fluorescent dye such as CR110 and TAMRA, and an oxazine-based fluorescent dye such as ATTO655 can be particularly preferably mentioned.

A method of labeling an antibody light-chain variable region polypeptide and an antibody heavy-chain variable region polypeptide with a fluorescent dye is not particularly limited. For example, a method of directly labeling a polypeptide by use of a functional group at both sides or a side chain thereof or indirectly labeling a polypeptide via a crosslinking agent; and a method of site-specifically labeling while synthesizing a polypeptide by use of an *in vitro* transcription/translation system can be used. As the labeling method using an *in vitro* transcription/translation system, an amber suppression method (Ellman J et al. (1991) Methods Enzymol. 202: 301-36), a C-terminal labeling method (Japanese Unexamined Patent Application Publication No. 2000-139468) and an N-terminal labeling method (U.S. Pat. No. 5,643,722, Olejnik et al. (2005) Methods 36: 252-260) are known. In the amber suppression method, DNA or mRNA is prepared by substituting a codon encoding an amino acid of the target site to be labeled with one of the termination codons, i.e., an amber codon, and then a protein is synthesized based on the DNA or the mRNA using an *in vitro* transcription/translation system. At this time, if a suppressor tRNA having a labeled non-natural amino acid bound thereto is added to a protein synthesis reaction solution, a protein having a labeled amino acid introduced in the amber codon substitution site can be synthesized. Furthermore, in the C-terminal labeling method, a protein having a label specifically introduced to the C-terminal can be synthesized by performing translation from DNA or mRNA to a protein in the *in vitro* transcription/translation system where a labeled puromycin is added in an optimal concentration.

The antigen-concentration measurement/detection kit may contain reagents and tools usually employed in immune measurement kits of this type, such as a buffer solution, a tube or plate for measurement and an antigen serving as a reference substance. The antigen-concentration measurement/detection kit of the present invention can be suitably used in the antigen-concentration measurement/detection method of the present invention.

In the step (a1-1) of the measurement method [I], after an antibody light-chain variable region polypeptide and an antibody heavy-chain variable region polypeptide labeled with a fluorescent dye are added to a solution such as a buffer solution and a physiological saline solution, a test substance is added and incubation is performed to form a ternary complex consisting of the antibody light-chain variable region polypeptide/the antibody heavy-chain variable region polypeptide labeled with a fluorescent dye/an antigen specifically recognized by the antibody, in the solution. Furthermore, in the step (a1-2) of the measurement method [II], after an antibody heavy-chain variable region polypeptide and an antibody light-chain variable region polypeptide labeled with a fluorescent dye are added to a solution such as a buffer solution and a physiological saline solution, a test substance is added and incubation is performed to form a ternary complex consisting of the antibody heavy-chain variable region polypeptide/the antibody light-chain variable region polypeptide labeled with a fluorescent dye/an antigen specifically recognized by the antibody, in the solution. Examples of the test substance include substances that possibly contain the target antigen to be measured, such as body fluid including serum, plasma, saliva and urine, a culture supernatant, a cell extract, a bacterial-cell extract and industrial effluent. Furthermore, the incubation conditions are not particularly limited as long as they are generally used for an antibody/antigen reaction. The temperature condition is for example, 1 to 30°C and preferably 18 to 25°C. The reaction time is, for example, 5 to 180 minutes and preferably 60 to 120 minutes. After completion of incubation, the solution can be subjected directly to the following step (b) without passing through a washing step, etc. This is a big feature of the antigen-concentration measurement/detection method.

In the step (b) of the measurement method [I] and the measurement method [II], the fluorescence intensity of a fluorescent dye in the solution can be measured by applying excitation light to the solution prepared in the step (a1-1) or (a1-2). A fluorescence measurement apparatus to be used for measurement is not particularly limited. For example, MF20/FluoroPoint-Light (manufactured by Olympus Corporation) and FMBIO-III (manufactured by Hitachi Software Engineering) can be preferably mentioned. Furthermore, in the measurement, it is preferable to measure the following solutions prepared as a negative control of the solution prepared in the step (a1-1):
1) a solution not containing an antibody light-chain variable region polypeptide and containing an antibody heavy-chain variable region polypeptide labeled with a fluorescent dye alone,
2) a solution not containing an antibody light-chain variable region polypeptide and containing an antibody heavy-chain variable region polypeptide labeled with a fluorescent dye and a test substance alone,
3) a solution containing an antibody light-chain variable region polypeptide and an antibody heavy-chain variable region polypeptide labeled with a fluorescent dye and not containing a test substance, and
4) a solution containing an antibody light-chain variable region polypeptide, a non-labeled antibody heavy-chain variable region polypeptide and a test substance.

It is also preferable to measure the following solutions prepared as a negative control of the solution prepared in the step (a1-2):
1) a solution not containing an antibody heavy-chain variable region polypeptide and containing an antibody light-chain variable region polypeptide labeled with a fluorescent dye alone,
2) a solution not containing an antibody heavy-chain variable region polypeptide and containing an antibody light-chain variable region polypeptide labeled with a fluorescent dye and a test substance alone,
3) a solution containing an antibody heavy-chain variable region polypeptide and an antibody light-chain variable region polypeptide labeled with a fluorescent dye and not containing a test substance, and
4) a solution containing an antibody heavy-chain variable region polypeptide, a non-labeled antibody light-chain variable region polypeptide and a test substance.

In the step (c) of the measurement method [I] and the measurement method [II], the level of an antigen contained in a test substance can be calculated from the measurement value of fluorescence intensity obtained in the step (b). To describe it more specifically, since antigen concentration in the solution prepared in the step (a1-1) or (a1-2) and fluorescence intensity measured in the step (b) have a positive correlation, the level of an antigen contained in a test substance can be obtained by measuring fluorescence intensity of a test substance containing an antigen having a known concentration, preparing a reference curve showing the relationship between the antigen concentration and the fluorescence intensity, and computationally obtaining the antigen concentration corresponding to the fluorescence-intensity measurement value of a test substance containing the antigen having an unknown concentration. Furthermore, "computationally obtaining the level of the antigen" in the step (c) includes also the case of automatically obtaining the level of an antigen based on e.g., a conversion equation, previously determined based on the reference curve.

In the step (a2-1) of the non-human animal detection method [I], an antibody light-chain variable region polypeptide and an antibody heavy-chain variable region polypeptide labeled with a fluorescent dye are administered to a non-human animal test subject to form a ternary complex consisting of the antibody light-chain variable region polypeptide/antibody heavy-chain variable region polypeptide labeled with a fluorescent dye/antigen specifically recognized by the antibody, in the non-human animal test subject. Furthermore, in the step (a2-2) of the non-human animal detection method [II], an antibody heavy-chain variable region polypeptide and an antibody light-chain variable region polypeptide labeled with a fluorescent dye are administered systemically or locally to a non-human animal test subject to form a ternary complex consisting of the antibody heavy-chain variable region polypeptide/antibody light-chain variable region polypeptide labeled with a fluorescent dye/antigen specifically recognized by the antibody, in the non-human animal test subject. The non-human animal test subject is not particularly limited as long as it is an animal except a human. For example, a mouse, a rat, a hamster, a monkey and a pig are preferably mentioned. Furthermore, the "administration" method can be appropriately selected from parenteral local injection methods such as intramuscular injection, intraperitoneal injection, intravenous injection, hypodermic injection, embedding and coating, and peroral administration methods.

In the step (b) of the non-human animal detection method [I] and the non-human animal detection method [II], the fluorescence of the fluorescent dye in a non-human animal test subject injected with the antibody light-chain variable region polypeptide and the antibody heavy-chain variable region polypeptide, either one of which is labeled with a fluorescent dye, in the above step (a2-1) or (a2-2), is detected directly from the animal in a non-invasive manner. Alternatively, the fluorescence of the fluorescent dye is detected from the tissue or a cell taken from the non-human animal test subject. The above "detection" method is not particularly limited as long as the fluorescence of the fluorescent dye can be two-dimensionally or three-dimensionally detected by irradiating a body, tissue or cell of a non-human animal test subject with excitation light. Furthermore, at the time of detection, an image showing the structure of a body, tissue or cell of a non-human animal test subject is preferably obtained by use of e.g., an endoscope, X-ray, CT, MRI, ultrasonic wave and a microscope, simultaneously with the detection.

In the step (c) of the non-human animal detection method [I] and the non-human animal detection method [II], the antigen in a non-human animal test subject is visualized from the detection results of fluorescence of the fluorescent dye obtained in the step (b). More specifically, since level of an antigen in a non-human animal test subject and the fluorescence intensity of fluorescence detected in the step (b) have a positive correlation, localization (position) of the antigen can be found by comparing image data showing the structure of the body, tissue or cell of the non-human animal test subject with a two-dimensional or three-dimensional image of fluorescence detected in the step (b). For example, when an endoscope is used for detection, the tissue of a non-human animal test subject is irradiated with visible light to form an observable visible-light image; at the same time, an antibody light-chain variable region polypeptide and an antibody heavy-chain variable region polypeptide, either one of which is labeled, is applied to the tissue, thereby allowing them to be contact with the tissue, and thereafter, excitation light for labeled fluorescent dye is irradiated to the tissue to form a fluorescent image. Localization of the antigen in the tissue can be found by comparing the visible-light image to the fluorescent image.

In the step (a3-1) of the *in vitro* detection method [I], an antibody light-chain variable region polypeptide, an antibody heavy-chain variable region polypeptide labeled with a fluorescent dye and an antigen in a test subject are incubated *in vitro* to form a ternary complex consisting of the antibody light-chain variable region polypeptide/the antibody heavy-chain variable region polypeptide labeled with a fluorescent dye/the antigen specifically recognized by the antibody. Furthermore, in the step (a3-2) of the *in vitro* detection method [II], an antibody heavy-chain variable region polypeptide, an antibody light-chain variable region polypeptide labeled with a fluorescent dye and an antigen in a test subject are incubated *in vitro* to form a ternary complex consisting of the antibody heavy-chain variable region polypeptide/the antibody light-chain variable region polypeptide labeled with a fluorescent dye/the antigen specifically recognized by the antibody. As the test subject, a test subject that possibly contains the target antigen to be measured such as a cultured cell, a tissue slice and tissue or cell obtained from a living body can be mentioned. Other than these, e.g., a cell extract blotted on e.g., a nitrocellulose film and a PVDF film can be mentioned. Furthermore, the incubation conditions are not particularly limited as long as they can be generally used for an antibody/antigen reaction. The temperature condition is, for example, 1 to 30°C and preferably 18 to 25°C. The reaction time is, for example, 5 to 180 minutes and preferably 60 to 120 minutes. After completion of incubation, the solution can be directly subjected to the following step (b) without subjecting to a washing step. This is a big feature of the antigen-concentration measurement/detection method.

In the step (b) of the *in vitro* detection method [I] and the *in vitro* detection method [II], the fluorescence of the fluorescent dye in a test subject incubated with the antibody light-chain variable region polypeptide and the antibody heavy-chain variable region polypeptide, either one of which is labeled with a fluorescent dye in the above step (a3-1) or (a3-2), is two-dimensionally or three-dimensionally detected. As the "detection" method, e.g., a fluorescence microscope and a fluorescence image analyzer can be mentioned.

In the step (c) of the *in vitro* detection method [I] and the *in vitro* detection method [II], the antigen in a test subject can be visualized from the detection results of fluorescence of the fluorescent dye obtained in the step (b). More specifically, since level of an antigen in a test subject and the fluorescence intensity of fluorescence detected in the step (b) have a positive correlation, localization (position) of the antigen can be found based on a two-dimensional or three-dimensional image of fluorescence detected in the step (b).

In Examples shown below, the present invention will be more specifically described in detail. The following Examples are described in order to explain the present invention.

### Examples

### [Example 1]

### 1. Establishment of homogenous fluorescence based immunoassay using VH and VL derived from anti-BGP antibody (Construction of anti-BGP antibody V-region gene expression vector)

To a DNA sequence encoding a heavy-chain variable region (VH: SEQ ID No:1) or a light-chain variable region (VL: SEQ ID No:2) of an antibody against human osteocalcin (human Bone Gla Protein: BGP), a DNA sequence of ProX^{™}tag (MSKQIEVNXSNET (X represents a fluorescent-labeled amino acid); SEQ ID No:3) containing an amber codon at the N-terminal was added. The resultant gene was inserted between the NcoI/HindIII sites of pIVEX2.3d vector (manufactured by Roche Diagnostics). The expression vector constructed is designed such that ProX^{™}tag (MSKQIEVNXSNET (X represents a fluorescent-labeled amino acid): SEQ ID No: 3) is added to the N-terminal of the inserted VH or VL, while His-tag is added to the C-terminal. Figure 1 schematically shows that a CR110-labeled anti-BGP antibody light-chain variable region polypeptide (CR110-VL), a TAMRA-labeled anti-BGP antibody heavy-chain variable region polypeptide (TAMRA-VH) and the complex (CR110-VL/TAMRA-VH) thereof. In the same manner, the TAG codon was substituted with a TTT codon, a fluorescent-labeled amino acid residue was substituted with a phenylalanine residue to prepare the wild-type VH and VL expression vectors.

Furthermore, the following expression vectors were also prepared: mutant VH (W33F, W36F, W47F, or W106F) expression vectors, each of which was prepared by substituting one of four tryptophan codons (TGG; Trp33, Trp36, Trp47, Trp106) contained in the above VH gene with phenylalanine codons (TTT); a spacer-added VH expression vector, which was prepared by adding a spacer (GGGSGGGS; SEQ ID No:4) between ProXtag and the N-terminal of the VH gene; a single-chain antibody (scFv) expression vector, which was prepared by linking the VH gene and the VL gene via a linker (LVTVSSGGGGSGGGGSGGGGS; SEQ ID No:5, or GGGGSGGGGSGGGGS; SEQ ID No:9); a single-chain antibody (scFv) expression vector having a spacer (GGGS, GGGSGGGS; SEQ ID No:4 or GGGSGGGSGGGS; SEQ ID No:10) between the ProX^{™}tag and the N-terminus of scFv; and an scFv expression vector having MX (X represents a fluorescent-labeled amino acid) at the N-terminus.

### (Preparation of labeled anti-BGP antibody V-region protein)

Using RTS100 *E.coli* Disulfide Kit (manufactured by Roche Diagnostics), a fluorescent-labeled amino acid was introduced into the V-region protein N-terminal region by a cell-free translation system. A reaction solution (50 µL) was prepared by adding 7 µL of amino acid mix, 1 µL of methionine, 7 µL of Reaction mix, 25 µL of activated *E. coli* lysate, 5 µL of plasmid DNA (500 ng) and 5 µL of fluorescent-labeled aminoacyl-tRNAamber (0.8 nmol). As fluorescent-labeled aminoacyl-tRNA (TAMRA-X-AF-tRNAamber, CR110-X-AF-tRNAamber, and ATT0655-X-AF-tRNAamber) for preparing a fluorescent-labeled protein, CloverDirect^{™} tRNA Reagents for Site-Derected Protein Functionalization (manufactured by ProteinExpress) was used. The reaction solution was allowed to react at 20°C, 600 rpm for 2 hours and thereafter at 4°C for 16 hours. After completion of the reaction, the reaction solution (1 µL) was subjected to SDS-PAGE (15%) and the expressed protein was observed by a fluorescence image analyzer (FMBIO-III; manufactured by Hitachi Software Engineering). Furthermore, Western blot was performed by use of an His-tag antibody to confirm the synthesis of target protein.

The V-region protein thus synthesized was purified by His-Spin Trap Column (manufactured by GE Healthcare). To the above reaction solution (50 µL), a Wash buffer (20 mM Phosphate buffer (pH 7.4)/0.5 M NaCl/60 mM imidazole/0.1% Polyoxyethylene (23) Lauryl Ether) was added up to a total volume of 400 µL. This was applied to His-Spin Trap Column. Incubation was performed at room temperature for 15 minutes and washing was performed with Wash buffer three times. Subsequently, elution was performed twice with 200 µL of Elute buffer (20 mM Phosphate buffer (pH 7.4)/0.5 M NaCl/0.5 M imidazole/0.1% Polyoxyethylene (23) Lauryl Ether). Furthermore, the eluate, the buffer of which was exchanged with PBS (+0.05% Tween20), was concentrated by use of UltraFree-0.5 centrifugal devices (manufactured by Millipore). After purification, the concentration of the sample was measured by SDS-PAGE and FCS (MF20; manufactured by Olympus Corporation).

### [Example 2]

### (Measurement of fluorescence spectra)

A sample was prepared by adding the TAMRA-labeled anti-BGP antibody VH protein and CR110-labeled anti-BGP antibody VL protein (1 µg/mL, 30 µL for each protein) prepared in Example 1 and 7-residue BGP C-terminal peptide (RRFYGPV; SEQ ID No:8) serving as an antigen and adjusted so as to have a total volume of 200 µL with PBS (+0.05% Tween20). After the mixture was allowed to stand still at 25°C for 90 minutes, fluorescence spectra were measured by a fluorescence spectrophotometer (FluoroMax-4; manufactured by HORIBA Jobin Yvon GmbH). The excitation wavelength to the mixture of CR110-VL and TAMRA-VH was set to 490 nm, and the excitation wavelength to TAMRA-VH was set to 550 nm. With respect to the mixture of CR110-VL and TAMRA-VH, a fluorescence intensity ratio, I_{A}/I_{D}, was calculated, in which I_{A} and I_{D} were defined as the fluorescence intensity at 575 nm and 525 nm, respectively. The dissociation constant (Kd) value was calculated based on the fluorescence intensity ratio (I_{A}/I_{D}) or by the curve-fitting of the fluorescence intensity of a maximum fluorescent wavelength. At this time, as statistical analysis software, a sigmoidal dose-response model of Graphpad Prism (manufactured by Graphpad) was used. After CR110-VL and TAMRA-VH were allowed to react in the presence of BGP peptides in different concentrations, fluorescence spectra were measured by use of excitation light of 490 nm. The results are shown in Figure 2. Changes of fluorescence intensity at 525 nm and 575 nm were measured, and the results are shown in Figure 3. A change of the fluorescence intensity ratio of 525 nm and 575 nm (F575/F525) was analyzed and the results are shown in Figure 4. A sample was prepared by adding a TAMRA-labeled anti-BGP antibody scFv protein (2 µg/mL, 25 µL) containing or not containing a spacer and a BGP peptide serving as an antigen, and adjusted so as to have a total volume of 200 µL with PBS (+0.05% Tween20, 0.2% BSA). Thereafter, the sample was allowed to stand still at 25°C for 70 minutes, and fluoroescence spectra were measured by use of a fluorescence spectrophotometer (FluoroMax-4; manufactured by HORIBA Jobin Yvon GmbH). The fluorescence intensity was calculated by curve-fitting. At this time, as statistical analysis software, a sigmoidal dose-response model of ImageJ software (http://rsbweb.nih.gov/ij/) was used. The measurement was performed at an excitation wavelength of 550 nm and a measurement wavelength of 580 nm.

### (Fluorescence intensity multiple distribution analysis)

A sample was prepared by adding the fluorescent-labeled VH protein and fluorescent-labeled VL protein (1 µg/mL, 7.5 µL for each protein) prepared in Example 1, or fluorescent-labeled scFv (1 µg/mL, 7.5 µL) and BGP peptide, and adjusted so as to have a volume of 50 µL with PBS (+0.05% Tween20). The resultant sample was added to a 384-well Glass Bottom Microplate (manufactured by Olympus Corporation) and incubated at 25°C for 90 minutes. Measurement in accordance with the fluorescence intensity multiple distribution analysis (FIDA) was performed at 25°C by use of MF20/FluoroPoint-Light (manufactured by Olympus Corporation). TAMRA and ATTO655 were excited by 543 nm- and 633 nm-laser, respectively. Data was obtained during 10 seconds per measurement and measurement was performed 10 times per sample. Based on these measurement values, an average value and a standard deviation were calculated.

### [Example 3]

### (Evaluation of binding activity of TAMRA-VH and CR110-VL to BGP peptide)

First, an attempt to establish an antibody/antigen binding activity evaluation system using a fluorescent resonance energy transfer method (FRET) was made. In the FRET measurement, CR110 and TAMRA were used as a donor and an acceptor, respectively. Since fluorescence of the donor (CR110) is sufficiently overlapping with absorption spectrum of the acceptor (TAMRA), they can be used as an FRET pair. Provided that an orientation factor (κ²) is set to be 2/3, the Foerster distance (R₀) is calculated as 62 Å, which value is suitable for detecting the intermolecular interaction of proteins. When an antigen is not present, the interaction between VL and VH is poor and thus FRET from CR110 to TAMAR does not occur; whereas, when an antigen is present, VH and VL form a ternary complex with an antigen. As a result, it is predicted to induce FRET from CR110 to TAMRA.

For the purpose of determining whether FRET from CR110 to TAMRA is detected or not, by binding CR110-VL and TAMRA-VH with an antigen interposed between them, the following experiment was performed. CR110-VL and TAMRA-VH or non-labeled VH were incubated together with BGP antigen peptides in different concentrations (1 to 10,000 ng), and a change of fluorescence intensity of CR110 was analyzed in accordance with the fluorescence intensity multiple distribution analysis (FIDA). Measurement and detection were performed by using a 488 nm laser as excitation light and a 510 to 560 nm fluorescence filter. The results are shown in Figure 5. When CR110-VL and TAMRA-VH were reacted, the fluorescence intensity of CR110 reduced in a BGP antigen peptide concentration-dependent manner. From the results, it was determined that FRET from CR110 to TAMRA was induced, as predicted, by forming a complex of CR110-VL and TAMRA-VH with an antigen peptide interposed between them. In contrast, unexpectedly, when CR110-VL was reacted with non-labeled VH, the fluorescence intensity of CR110 increased in a BGP antigen peptide concentration-dependent manner. From the results, the possibility that VL serves as a quencher to CR110; more specifically, following possibility was predicted. When CR110-VL is present alone, fluorescence of CR110 is quenched by VL; however, when the ternary complex of CR110-VL, VH and an antigen peptide is formed, the quenching effect is eliminated.

For the purpose of determining whether such a quenching effect by VL is observed also in VH, the following experiment was performed. TAMRA-VH and either CR110-VL or non-labeled VL were incubated together with BGP antigen peptides in different concentrations (1 to 10,000 ng), and the change of fluorescence intensity of TAMRA was analyzed in accordance with the fluorescence intensity multiple distribution analysis (FIDA). Measurement and detection were performed by using a 543 nm laser as excitation light and a 560 to 620 nm fluorescence filter. The results are shown in Figure 6. When TAMRA-VH and CR110-VL were reacted, the fluorescence intensity of TAMRA increased in a BGP antigen peptide concentration-dependent manner. Similarly, when TAMRA-VH and non-labeled VL were reacted, the fluorescence intensity of TAMRA-VH also increased in a BGP antigen peptide concentration-dependent manner. From the results, similarly to the case of VL, the possibility that VH also serves as a quencher to TAMRA, more specifically, when TAMRA-VH is present alone, the fluorescence of TAMRA is quenched; however, when a ternary complex of TAMRA-VH, VL and an antigen peptide is formed, the quenching effect is eliminated, was predicted. From the results above, it was suggested that VH and VL have a quenching effect to a fluorescent dye, and furthermore the effect was eliminated upon formation of a ternary complex of VH/VL/antigen (Figure 9).

### [Example 4]

### (Establishment of homogenous fluorescence based immunoassay of antigen concentration using quenching phenomenon)

The inventors assumed that a novel immunoassay method may be established if a quenching phenomenon demonstrated in Example 3 is used. Based on the assumption, the following experiment was performed. TAMRA-VH and BGP peptides in different concentrations were reacted in the presence or absence of non-labeled VL, and fluorescence intensity was measured by use of an He-Ne laser of 543 nm. The results are shown in Figure 7. In the presence of VL, the fluorescence intensity of TAMRA-VH increased in a BGP peptide concentration-dependent manner. In contrast, in the absence of VL, the fluorescence intensity of TAMRA-VH remained low in any concentration of BGP peptides used in the reaction. Furthermore, the fluorescence intensity ratio (+VL/-VL) of TAMRA-VH, in the presence of VL relative to TAMRA-VH in the absence of VL, was analyzed. As a result, the dissociation constant Kd was calculated as 1.2 × 10⁻⁷ [M] (Figure 8). From the above results, it was demonstrated that a genuine novel homogenous fluorescence based immunoassay was successfully established using quenching phenomenon by VH and VL proteins.

### [Example 5]

### (Study on quenching effect by Trp in VH)

From the results of Example 3, it was demonstrated that the increase of TAMRA fluorescence intensity is higher than the decrease of CR110 fluorescence intensity, per titer of antigen. TAMRA is a rhodamine-based dye, and the studies so far made have reported that a rhodamine-based dye is quenched by an amino acid such as tryptophan (Trp). Hence, the inventors predicted that a Trp residue present in VH is involved in the quenching of TAMRA and postulated that when TAMRA-VH is present alone, fluorescence of TAMRA is quenched by the Trp residues present in the vicinity thereof; however, if TAMRA-VH forms a complex with VL and an antigen, the positional relationship between TAMRA and Trp may be changed to negate quenching. As shown in Figure 9, VH has 4 Trp residues (Trp33, Trp36, Trp47, Trp106). From the analysis of predicted molecular model, it is estimated that Trp33, Trp36 and Trp106 are involved in hydrophobic interaction with VL; whereas Trp33 is involved in interaction with a BGP peptide. For the purpose for determining whether these Trp residues have an effect upon quenching, the following experiment was performed by using four types of mutant VHs, in which each Trp was substituted with Phe.

Either the wild-type or mutant anti-BGP antibody heavy-chain variable region polypeptide (W33F, W36F, W47F, or W106F) and VL were reacted in the presence of BGP peptides in different concentrations, and fluorescence intensity was measured by use of an He-Ne laser of 543 nm. The results are shown in Figure 10 and Table 1. When the fluorescence intensity of a mutant fluorescent-labeled VH alone was measured, compared to the wild-type (WT), the fluorescence values of W106F and W36F exhibited an increase of 31% and 29%, respectively. The fluorescence of W47F exhibited an increase of 11%. In contrast, W33F exhibits a decrease of 9%. From these results, it was demonstrated that Trp36, Trp47 and Trp106 are mainly involved in the quenching of TAMRA fluorescence. Furthermore, in the cases of W33F, W36F, and W106F, if they are reacted with VL and a BGP peptide, fluorescence increases 1.5 times, 1.3 times and 1.5 times in an antigen-concentration dependent manner, respectively. The resultant fact that elimination of antigen-dependent quenching is attenuated by substitution of Trp by Phe suggests that Trp33, Trp36 and Trp106 are partly involved in quenching. In contrast, in the case of W47F, if it is reacted with a BGP peptide and VL, no increase of fluorescence was observed. Analysis results (Figure 11) of diffusion time by FCS measurement show that binding activity of an antibody disappears by the mutation of Trp47. From this, it was demonstrated that Trp47 of VH is essential for binding between an antibody and an antigen.

From the above results, it was determined that Trp33 and Trp106 are critical Trp residues for quenching in view of two points: increasing fluorescence of a fluorescent-labeled VH alone and reducing an increase of fluorescence in an antigen-concentration dependent manner. Furthermore, involvement of Trp47 in quenching in an antigen-concentration dependent manner is not elucidated; however, it was elucidated that Trp47 is a very important site for formation of a VH and VL complex via an antigen. Note that Trp106 in the amino acid sequence of VH corresponds to the 103rd position according to the Kabat numbering system (Kabat, E. et al., "Sequences of proteins of immunological interest, 5th edn.," U.S. Department of Health and Human Service, Public Service, National Institute of Health, Washington, DC, 1991).

**Table 1. Relative Fluorescence Quenching of TAMRA-labeled VH proteins.**

| V_{H} proteins | Fluorescence intensity (*I*₀)*¹ | Fluorescence intensity (*I*_{H})*² | Fluorescence intensity (*I*_{HP})*³ | Increased amount of fluorescence⁴ (*I*₀/*I*_{WT}) | Amount of change in fluorescence⁵ (*I*_{HP}/*I*_{H}) | K_{d}*⁶ |
|---|---|---|---|---|---|---|
| WT | 24.7 | 25.6 | 47.1 | 1.00 | 1.84 | 1.2×10⁻⁷ |
| W33F | 21.8 | 23.7 | 34.8 | 0.88 | 1.47 | 1.6×10⁻⁷ |
| W36F | 31.8 | 31.5 | 41.4 | 1.29 | 1.31 | 7.2×10⁻⁸ |
| W47F | 26.7 | 27.4 | 27.6 | 1.08 | 1.01 | ND |
| W106F | 30.8 | 31.9 | 46.7 | 1.25 | 1.46 | 1.4×10⁻⁵ |

| | | | | | | |
|---|---|---|---|---|---|---|
| *1 Fluorescence intensity in the absence of (*I*₀) = VL) or BGP pep. (fluorescence intensity of fluorescent labeled VH alone) *2 Fluorescence intensity in the presence of (*I*_{H}) = VL and in absence of BGP pep. *3 Fluorescence intensity in the presence of (*I*_{HP}) = VL and BGP pep. (concentration of BGP pep. herein is 10,000 ng/mL) *4 Increased amount of fluorescence = rate of fluorescence intensity *I*₀ of each protein relative to fluorescence intensity *I*₀ of WT (represented by *I*_{wt}) *5 Amount of change in fluorescence = Amount of fluorescence increased in an antigen-concentration dependent manner *6 Dissociation constant | | | | | | |

### [Example 6]

### (Study on quenching effect by addition of oxazine-based fluorescent dye and spacer)

Effect of a fluorescent dye and a spacer on sensitivity of homogenous fluorescence based immunoassay was studied. Efficiency of quenching greatly changes depending on the type of fluorescent dye. It has been reported that oxazine-based dyes are more effectively quenched than rhodamine-based dyes. Hence, the inventors prepared ATT0655-VH using an oxazine-based fluorescent dye ATTO655 as a label, and the same experiment as in Example 4 was performed. Furthermore, an ATTO655-VH (+spacer) was prepared by inserting GGGSGGGS (SEQ ID No:4) as a spacer between ATTO655 and VH, and the effect of the spacer on the quenching effect was studied. Figure 12 shows a predicted three-dimensional structure model, a complex (Fluorescent-labeled BGP-VH/VL) of a spacer-added fluorescent-labeled anti-BGP antibody heavy-chain variable region polypeptide (Fluorescent-labeled BGP-VH) and an anti-BGP antibody light-chain variable region polypeptide (BGP-VL).

The same experiment as in Example 4 was performed by using ATT0655-VH with a spacer added thereto or without it. The results are shown in Figure 13. In each case, the fluorescence intensity of ATT0655-VH increased in a BGP peptide concentration-dependent manner. Furthermore, the intensity of fluorescence obtained from ATT0655-VH having no spacer added thereto was three times as large as that of the case where TAMRA-VH was used. Furthermore, it was revealed that fluorescence intensity increases about twice by adding a spacer, compared to the case where no spacer is added. This is considered because the distance between quencher Trp and a fluorescent dye is reduced by adding a GGGS spacer, which has an effect on an increase of fluorescence intensity.

Furthermore, the fluorescence intensity ratio (+VL/- VL) of ATT0655-VH in the presence of VL to ATT0655-VH in the absence of VL, was analyzed, and the results are shown in Figure 14. In the case of no spacer added, a dissociation constant Kd was calculated as 8.4 × 10⁻⁸ [M]; whereas in the case of a spacer added, the dissociation constant Kd was 1.8 × 10⁻⁷ [M]. Since it is considered that the sensitivity of the measurement system increases as the dissociation constant increases, the above results mean that a measurement system having further higher sensitivity was successfully established by providing a spacer between a fluorescent dye and VH.

### [Example 7]

### (Establishment of homogenous fluorescence based immunoassay using single-chain antibody)

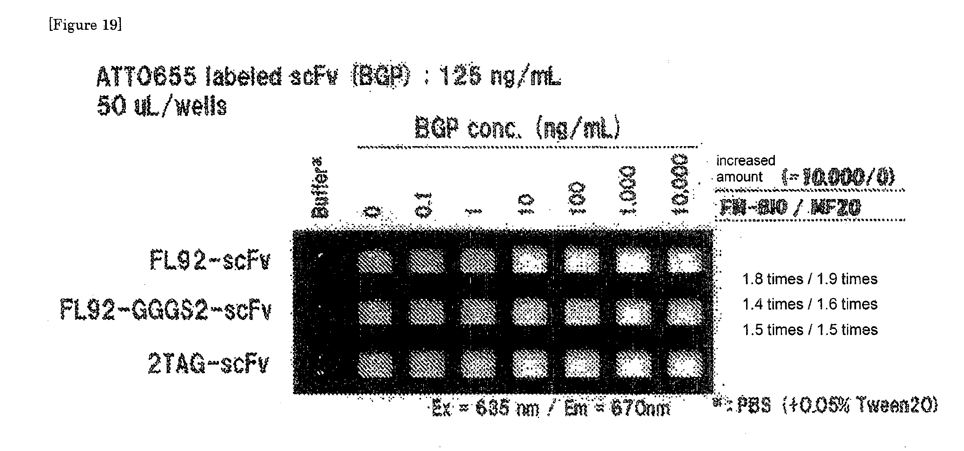

For the purpose of establishing a homogenous fluorescence based immunoassay using single-chain antibody (scFv) in which VH and VL were linked by a linker consisting of the amino acid sequence represented by SEQ ID No:5 or 9, the following experiment was performed. A predicted three-dimensional structure model of a fluorescent-labeled single-chain antibody formed by linking a fluorescent-labeled antibody heavy-chain variable region polypeptide and an antibody light-chain variable region polypeptide is shown in Figure 15, and the primary structure thereof is shown in Figure 16. Similarly to the case where peptide fragments of VH and VL were used, also in the case of a fluorescent-labeled scFv, it was observed that fluorescence intensity increased in a BGP peptide concentration-dependent manner. The results of ATT0655-labeled anti-BGP antibody scFv linked by the linker represented by SEQ ID No:5 are shown in Figures 17 to 22; whereas, the results of TAMRA-labeled anti-BGP antibody scFv linked by the linker represented by SEQ ID No:9 are shown in Figure 23.

### [Example 8]

### (Fluorescence immunoassay method in the human plasma)

A sample was prepared by adding a TAMRA-labeled anti-BGP antibody scFv protein (2 µg/mL, 6.25 µL) and a BGP peptide serving as an antigen so as to contain 50% human plasma, and adjusted so as to have a total volume of 50 µl with PBS (+0.05% Tween20, 0.2% BSA). Thereafter, the sample was allowed to stand still at 25°C for 90 minutes and then observed by a fluorescence image analyzer (FMBIO-III; manufactured by Hitachi Software Engineering). Measurement was performed at an excitation wavelength of 532 nm and at a measurement wavelength of 580 nm. The results are shown in Figure 24. Even in the sample containing 50% human plasma, it was observed that fluorescence intensity increased in a BGP peptide concentration-dependent manner.

### [Example 9]

### 2. Establishment of homogenous fluorescence based immunoassay using VH and VL derived from anti-BPA antibody (Construction of anti-BPA antibody derived V-region gene expression vector)

To a gene encoding an anti-bisphenol A (BPA) antibody VH (SEQ ID No:6), a DNA sequence of ProX™tag (MSKQIEVNXSNET (X represents a fluorescent-labeled amino acid); SEQ ID No:3) containing an amber codon at the N-terminal was added. The resultant gene was inserted between the NcoI-HindIII sites of pIVEX2.3d vector (manufactured by Roche Diagnostics). The expression vector thus constructed is designed such that ProX™tag (MSKQIEVNXSNET (X represents a fluorescent-labeled amino acid); SEQ ID No:3) containing an amber codon is added to the N-terminal of the inserted VH and His-tag is added to the C-terminal. Furthermore, to a gene encoding an anti-BPA antibody VL (SEQ ID No:7), a DNA sequence in which amino acid X of the N-terminal ProX™tag is substituted with F was added in the same manner. The resultant gene was integrated in an NcoI-HindIII site of pIVEX2.3d vector (manufactured by Roche Diagnostics). The expression vector thus constructed is designed such that the sequence in which amino acid X of ProX™tag substituted with F is added to the N-terminal of the VL inserted and His-tag is added to the C-terminal. Moreover, a single-chain antibody (scFv) expression vector in a VH gene and a VL gene are bound by a linker (GGGGSGGGGSGGGGS; SEQ ID No:9) and three types of single-chain antibody (scFv) expression vectors having spacers: GGGSGGGS; SEQ ID No:4, GGGSGGGSGGGS; SEQ ID No:10 and GGGSGGGSGGGSGGGSGGGS; SEQ ID No:11, respectively, between ProX™tag and the N-terminal of scFv, were simultaneously prepared.

### (Preparation of fluorescent-labeled protein in cell-free translation system)

Using RTS100 E.coli Disulfide Kit (manufactured by Roche Diagnostics), a fluorescent-labeled amino acid was introduced into the N-terminal region of a V-region protein, by a cell-free translation system. A reaction solution (50 µL) was prepared by adding 7 µL of amino acid mix, 1 µL of methionine, 7 µL of Reaction mix, 25 µL of activated *E.coli* lysate, 5 µL of plasmid DNA (500 ng) and 5 µL of ATTO655-X-AF-tRNAamber (0.8 nmol). As ATT0655-X-AF-tRNAamber for preparing a fluorescent-labeled protein, CloverDirect^{™} tRNA Reagents for Site-Directed Protein Functionalization (manufactured by ProteinExpress) was used. The reaction solution was allowed to react at 20°C, 600 rpm for 2 hours and thereafter at 4°C for 16 hours. After completion of the reaction, the reaction solution (1 µL) was subjected to SDS-PAGE (15%) and protein expression was observed by a fluorescence image analyzer (FMBIO-III; manufactured by Hitachi Software Engineering). Furthermore, Western blot was performed by use of an His-tag antibody to confirm the synthesis of a target protein.

The V-region protein thus synthesized was purified by His-Spin Trap Column (manufactured by GE Healthcare). To the above reaction solution (50 µL), a Wash buffer (20 mM Phosphate buffer (pH 7.4)/0.5 M NaCl/60 mM imidazole/0.1% Polyoxyethylene (23) Lauryl Ether) was added to bring the mixture to a total volume of 400 µL. This was applied to His-Spin Trap Column. Incubation was performed at room temperature for 15 minutes and washing was performed with Wash buffer three times. Subsequently; elution was performed with 200 µL of Elution buffer (20 mM Phosphate buffer (pH 7.4)/0.5 M NaCl/0.5 M imidazole/0.1% Polyoxyethylene (23) Lauryl Ether), twice. Furthermore, the eluate, the buffer of which was exchanged with PBS (+0.05% Tween20), was concentrated by use of UltraFree-0.5 centrifugal devices (manufactured by Millipore). The concentration of the sample was measured by SDS-PAGE and FCS (MF20; manufactured by Olympus Corporation).

### [Example 10]

### (Measurement of fluorescence spectrum)

A sample was prepared by adding the ATT0655-labeled anti-BPA antibody VH protein prepared in Example 9, a non-labeled anti-BPA antibody VL protein (1 µg/mL, 7.5 µL, respectively) and BPA serving as an antigen so as to bring a total volume of 50 µl of a 10% MeOH solution in PBS (+0.05% Tween20). After the mixture was allowed to stand still at 25°C for 90 minutes, it was observed by a fluorescence image analyzer (FMBIO-III; manufactured by Hitachi Software Engineering). Measurement was performed at an excitation wavelength of 635 nm and at a measurement wavelength of 670 nm. A dissociation constant (Kd) value was calculated by the curve-fitting of a fluorescence measurement value. At this time, as statistical analysis software, a sigmoidal dose-response model of Graphpad Prism (manufactured by Graphpad) was used.

A sample was prepared by adding a TAMRA-labeled anti-BGP antibody scFv protein (2 µg/mL, 25 µL) containing or not containing a spacer and BGP serving as an antigen and adjusted so as to have a total volume of 200 µL with PBS (+0.05% Tween20, 0.2% BSA, 1% MeOH). Thereafter the sample was allowed to stand still at 25°C for 10 minutes and then a fluorescence spectrum was measured by use of a fluorescence spectrophotometer (FluoroMax-4; manufactured by HORIBA Jobin Yvon GmbH). The fluorescence intensity was calculated by curve-fitting. At this time, as statistical analysis software, a sigmoidal dose-response model of ImageJ software (http://rsbweb.nih.gov/ij/), was used. The measurement was performed at an excitation wavelength of 550 nm and a measurement wavelength of 580 nm.

### [Example 11]

### (Establishment of homogenous fluorescence based immunoassay using labeled V-region protein derived from anti-BPA antibody

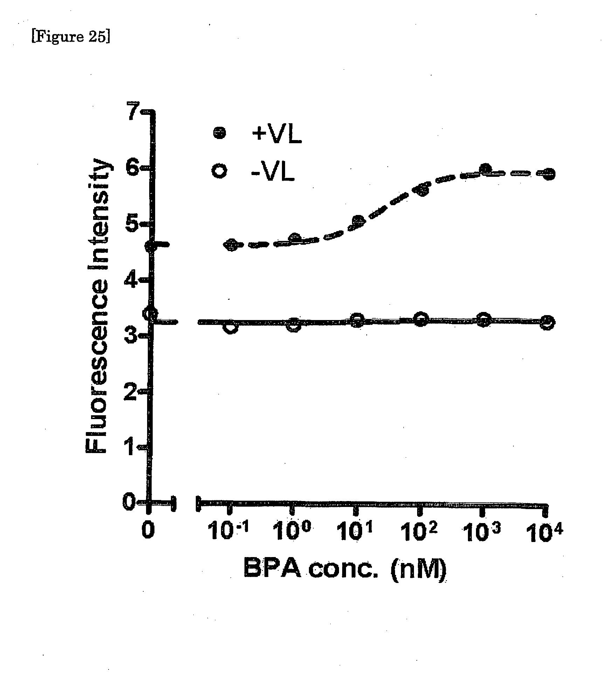

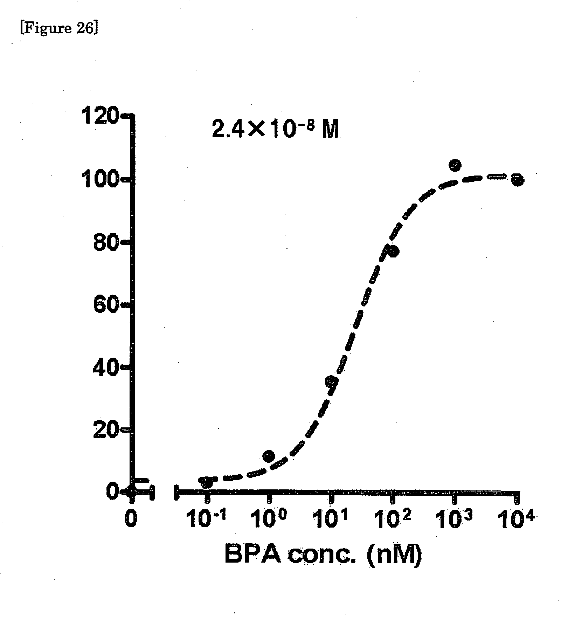

For the purpose of establishing a homogenous fluorescence based immunoassay using ATT0655-VH and VL without fluorescent-label, the following experiment was performed. ATT0655-VH and BPA in different concentrations were allowed to react in the presence or absence of non-labeled VL, and fluorescence intensity was measured (Figure 25). In the presence of VL, the fluorescence intensity of ATT0655-VH increased in a BPA concentration dependent manner. In contrast, in the absence of VL, the fluorescence intensity of ATT0655-VH remained low in the case where any concentration of BPA was used in the reaction. Furthermore, the fluorescence intensity ratio of ATT0655-VH (+VL/-VL) in the presence of VL to ATT0655-VH in the absence of VL was analyzed. As a result, a dissociation constant (Kd) was calculated as 2.4 × 10⁻⁸ [M] (Figure 26). Furthermore, for the purpose of establishing a fluorescence immunoassay method using a single-chain antibody (scFv) similarly to Example 7, the following experiment was performed. Also in the case where a TAMRA-labeled anti-BPA antibody scFv was used, it was observed that fluorescence intensity increased in a BPA concentration dependent manner (Figure 27), similarly to the case where each of VH and VL peptide fragments was used.

### [Example 12]

### 3. Establishment of homogenous fluorescence-based immunoassay using single-chain antibodies derived from various antibodies

### (Construction of anti-HEL antibody, anti-estradiol antibody, anti-SA antibody-derived V-region gene expression vector)

Expression vectors were constructed by inserting a DNA sequence, which has a ProX™tag (MSKQIEVNXSNET (X represents a fluorescent-labeled amino acid); SEQ ID No:3) containing an amber codon at the N-terminal of a DNA sequence of a single-chain antibody (scFv), an His-tag at the C-terminal, and a spacer (GGGSGGGS; SEQ ID No:4) between ProX™tag and the N-terminal of the scFv, between the NcoI and HindIII sites of pIVEX2.3d vector (manufactured by Roche Diagnostics). A DNA sequence of each single-chain antibody (scFv) is as follows:
an anti-chicken egg lysozyme (HEL) antibody scFv has a sequence formed by connecting a VH sequence (SEQ ID No: 12) and a VL sequence (SEQ ID No: 13) of an anti-HEL antibody in this order, by a linker sequence (GGGGSGGGGSGGGGS; SEQ ID No:9);
Estradiol (estradiol) antibody scFv has a sequence formed by connecting a VH sequence (SEQ ID No:14) and a VL sequence (SEQ ID No:15) of an anti-estradiol antibody in this order, by a linker sequence (GGGGSGGGGSGGGGS; SEQ ID No:9); and
SA (Serum Albumin) antibody scFv has a sequence formed by connecting a VH sequence (SEQ ID No:16) and a VL sequence (SEQ ID No:17) of an anti-SA antibody in this order, by a linker sequence (GGGGSGGGGSGGGGS; SEQ ID No:9).

### (Preparation of fluorescent-labeled anti-HEL antibody, anti-estradiol antibody, anti-SA antibody V-region protein)

Using RTS100 *E.coli* Disulfide Kit (manufactured by Roche Diagnostics), a fluorescent-labeled amino acid was introduced to the V-region protein N-terminal region by a cell-free translation system. A reaction solution (50 µL) was prepared by adding 7 µL of amino acid mix, 1 µL of methionine, 7 µL of reaction mix, 25 µL of activated E.coli lysate, 5 µL of plasmid DNA (500 ng) and 5 µL of fluorescent-labeled aminoacyl-tRNAamber (0.8 nmol). As the fluorescent-labeled aminoacyl-tRNA (TAMRA-X-AF-tRNAamber) for preparing a fluorescent labeled protein, CloverDirect^{™} tRNA Reagents for Site-Directed Protein Functionalization (manufactured by ProteinExpress) was used. The reaction solution was allowed to react at 20°C, 600 rpm for 2 hours and thereafter at 4°C for 16 hours. After completion of the reaction, the reaction solution (1 µL) was subjected to SDS-PAGE (15%), and protein expression was observed by a fluorescence image analyzer (FMBIO-III; manufactured by Hitachi Software Engineering). Furthermore, Western blot was performed by use of an His-tag antibody to confirm the synthesis of a target protein.

The V-region protein thus synthesized was purified by His-Spin Trap Column (manufactured by GE Healthcare). To the above reaction solution (50 µL), a Wash buffer (20 mM Phosphate buffer (pH 7.4)/0.5 M NaCl/60 mM imidazole/0.1% Polyoxyethylene (23) Lauryl Ether) was added to bring a total volume to 400 µL. This was applied to His-Spin Trap Column. Incubation was performed at room temperature for 15 minutes and washing was performed with Wash buffer three times. Subsequently, elution was performed with 200 µL of Elution buffer (20 mM Phosphate buffer (pH 7.4)/0.5 M NaCl/0.5 M imidazole/0.1% Polyoxyethylene (23) Lauryl Ether), twice. Furthermore, the eluate, the buffer of which was exchanged with PBS (+0.05% Tween20), was concentrated by use of UltraFree-0.5 centrifugal devices (manufactured by Millipore). After purification, the concentration of the sample was measured by SDS-PAGE and FCS (MF20; manufactured by Olympus Corporation).

### [Example 13]

### (Measurement of fluorescence spectrum)

A sample was prepared by adding a TAMRA-labeled anti-HEL antibody scFv protein (2 µg/mL, 25 µL) and an HEL protein serving as an antigen and adjusted so as to have a total volume of 200 µL with PBS (+0.05% Tween20, 1% BSA). A sample was prepared by adding a TAMRA-labeled anti-estradiol antibody scFv protein (2 µg/mL, 25 µL) and estradiol serving as an antigen and adjusted so as to have a total volume of 200 µL with PBS (+0.05% Tween20, 1% BSA). A sample was prepared by adding a TAMRA-labeled anti-SA antibody scFv protein (2 µg/mL, 25 µL) and BSA (bovine serum albumin) or HSA (human serum albumin) serving as an antigen and adjusted so as to have a total volume of 200 µL with PBS (+0.05% Tween20, 0.2% gelatin). After the samples were allowed to stand still at 25°C for 5 minutes, fluorescence spectra were measured by a fluorescence spectrophotometer (FluoroMax-4; manufactured by HORIBA Jobin Yvon GmbH) and the fluorescence intensity was calculated by curve fitting. At this time, as statistical analysis software, a sigmoidal dose-response model of ImageJ software (http://rsbweb.nih.gov/ij/), was used. The measurement was performed at an excitation wavelength of 550 nm and a measurement wavelength of 580 nm. As a result, it was observed that fluorescence intensity increased in an antigen-concentration dependent manner (Figures 28 to 30). As described above, it was demonstrated that fluorescence immunoassay method can be performed by using various types of fluorescent-labeled single-chain antibodies (scFv).

### [Example 14]

### 4. Conservation of Trp residue in mouse antibody VH

As shown in Example 4, Figure 9 and Table 1, it was found that the 33rd, 36th and 106th Trp (according to the Kabat numbering system) of the VH amino acid sequence have an important role in quenching a fluorescent-dye labeled to an anti-BGP antibody. It was further found that the 47th Trp is essential for binding between an antibody (VH and VL) to an antigen (Note that Trp106 in the VH amino acid sequence corresponds to the 103rd position according to the Kabat numbering system). Hence, the present inventors investigated whether these tryptophan residues are conserved in mouse antibody VH-regions other than the anti-BGP antibody. Amino acid residue distribution of mouse antibodies was analyzed by use of Abysis database (Dr. Andrew C.R. Martin's Group; http://www.bioinf.org.uk/abs/reference.html). Furthermore, residue numbers of each antibody residue according to the Kabat sequence notational system were investigated based on AbCheck (Dr. Andrew C.R. Martin's Group; Martin, A.C.R. Accessing the Kabat Antibody Sequence Database by Computer PROTEINS: Structure, Function and Genetics, 25 (1996), 130-133; http://www.bioinf.org.uk/abs/seqtest.html). As is apparent from the results shown in Figures 31 to 35, the conservation rates of four Trp residues in the mouse antibody VH-region were as follows: Trp33: 40%; Trp36: 98%; Trp47: 94%; and Trp103: 95%. These results show that the four Trp residues of VH, which are critical for carrying out a homogenous fluorescence based immunoassay, are conserved in many mouse-antibody VH sequences.

The positions of tryptophan residues contained in VH (SEQ ID No:1) and VL (SEQ ID No:2) of an anti-BGP antibody and VH (SEQ ID No:6) and VL (SEQ ID No:7) of anti-BPA antibody used in aforementioned Examples were numbered in accordance with the Kabat sequence notational system. The results are shown in Table 2. Furthermore, the positions of tryptophan residues contained in anti-BGP antibody scFv, anti-BPA antibody scFv, anti-HEL antibody scFv, anti-SA antibody scFv and anti-estradiol antibody scFv were numbered in accordance with the Kabat sequence notational system. The results are shown in Table 3.

| V_{H} | Kabat sequence notational system | Trp33 | Trp36 | Trp47 | Trp103 |
|---|---|---|---|---|---|
| | BGP | Trp33 | Trp36 | Trp47 | Trp106 |
| | BPA | Trp35 | Trp37 | Trp48 | Trp109 |
| | | | | | |

| V_{L} | Kabat sequence notational system | Trp35 | | | |
|---|---|---|---|---|---|
| | BGP | Trp40 | | | |
| | BPA | Trp39 | | | |

| Residue number* | | | | | | | | | | | | | | | | | | | | | |
|---|---|---|---|---|---|---|---|---|---|---|---|---|---|---|---|---|---|---|---|---|---|
| | CDRH1 | | | | | | | CDRH3 | | | | | | | | | | CDRL3 | | | |
| ... | H33 | H34 | H35 | H36 | ... | H47 | ... | H95 | ... | H103 | ... | L35 | ... | L47 | ... | L91 | ... | L92 | ... | L94 | ... |
| αBGP | **W** | I | H | **W** | | **W** | | S | | **W** | | **W** | | L | | T | | T | | V | |
| αBisphenolA | Y | Y | **W** | **W** | | **W** | | V | | **W** | | **W** | | I | | S | | **W** | | I | |
| αHEL | Y | **W** | S | **W** | | Y | | **W** | | **W** | | **W** | | L | | S | | N | | **W** | |
| αBSA | A | M | S | **W** | | **W** | | S | | **W** | | **W** | | L | | A | | D | | S | |
| αEstradiol | T | I | H | **W** | | **W** | | Y | | **W** | | **W** | | **W** | | **W** | | S | | Y | |

### Industrial Applicability

It is possible to provide a homogenous fluorescence based immunoassay using an antibody (fragment) labeled with a fluorescent dye with reference to elimination of quenching of a fluorescent dye. In the homogenous fluorescence based immunoassay, the concentration of a target substance can be measured by directly monitoring the fluorescence intensity of a mixture solution having an antibody and a test substance mixed therein without immobilization and washing of an antibody or an antigen. Thus, it is presumed that a low-molecular compound can be quickly and easily detected. Furthermore, since Trp residues of an antibody VH-region having an effect on quenching are conserved in many types of antibodies, the homogenous fluorescence based immunoassay can be used in measurement of various antigen concentrations.

## Claims

1. An antigen-concentration measurement/detection kit comprising an antibody light-chain variable region polypeptide and an antibody heavy-chain variable region polypeptide, either one of which is labeled with a fluorescent dye that is quenched in the state where it is labeled to the antibody heavy-chain variable region polypeptide or the antibody light-chain variable region polypeptide, wherein the quench is eliminated when the antibody heavy-chain variable region polypeptide and the antibody light-chain variable region polypeptide form a complex via an antigen to increase a fluorescence intensity, and wherein the kit enables a measurement of antigen concentration or visualization of the antigen with reference to a positive correlation between the concentration of the antigen in a liquid phase and the fluorescence intensity of the fluorescent dye.

2. The antigen-concentration measurement/detection kit according to Claim 1, wherein the antibody heavy-chain variable region polypeptide and the antibody light-chain variable region polypeptide are linked to form a single-chain antibody.

3. The antigen-concentration measurement/detection kit according to Claim 1 or 2, wherein the fluorescent dye is a rhodamine-based fluorescent dye or an oxazine-based fluorescent dye.

4. The antigen-concentration measurement/detection kit according to Claim 3, wherein the fluorescent dye is CR110, TAMRA or ATTO655.

5. The antigen-concentration measurement/detection kit according to any one of Claims 1 to 4, wherein the antibody heavy-chain variable region polypeptide includes a polypeptide consisting of the amino acid sequence represented by SEQ ID No:1 and the antibody light-chain variable region polypeptide includes a polypeptide consisting of the amino acid sequence represented by SEQ ID No:2.

6. The antigen-concentration measurement/detection kit according to any one of Claims 1 to 4, wherein the antibody heavy-chain variable region polypeptide includes a polypeptide consisting of the amino acid sequence represented by SEQ ID No:6 and the antibody light-chain variable region polypeptide includes a polypeptide consisting of the amino acid sequence represented by SEQ ID No:7.

7. An antigen-concentration measurement/detection method comprising the following steps (a) to (c) sequentially in this order:
(a) bringing an antibody light-chain variable region polypeptide and an antibody heavy-chain variable region polypeptide labeled with a fluorescent dye that is quenched in the state where it is labeled to the antibody light-chain variable region polypeptide; or an antibody heavy-chain variable region polypeptide and an antibody light-chain variable region polypeptide labeled with a fluorescent dye that is quenched in the state where it is labeled to the antibody heavy-chain variable region polypeptide into contact with
(a1) an antigen in a test substance in a liquid phase, or
(a2) an antigen in a sample of a non-human animal test subject administered with the antibody light-chain variable region polypeptide and the antibody heavy-chain variable region polypeptide labeled with a fluorescent dye; or the antibody heavy-chain variable region polypeptide and the antibody light-chain variable region polypeptide labeled with a fluorescent dye, or
(a3) an antigen of a test subject in vitro;
(b) measuring the fluorescence intensity of the fluorescent dye in the case of (a1) and detecting fluorescence of the fluorescent dye in the cases of (a2) and (a3); and
(c) computationally obtaining level of the antigen contained in the test substance in the case of (a1) and visualizing the antigen contained in the test subject in the cases of (a2) and (a3), with reference to the positive correlation between the concentration of the antigen in the liquid phase and the fluorescence intensity of the fluorescent dye, wherein the quench is eliminated when the antibody heavy-chain variable region polypeptide and the antibody light-chain variable region polypeptide form a complex via the antigen to increase the fluorescence intensity.

8. The antigen-concentration measurement/detection method according to Claim 7, wherein the antibody heavy-chain variable region polypeptide and the antibody light-chain variable region polypeptide are linked to form a single-chain antibody.

9. The antigen-concentration measurement/detection method according to Claim 7 or 8, wherein the fluorescent dye is a rhodamine-based fluorescent dye or an oxazine-based fluorescent dye.

10. The antigen-concentration measurement/detection method according to Claim 9, wherein the fluorescent dye is CR110, TAMRA or ATTO655.

11. The antigen-concentration measurement/detection method according to any one of Claims 7 to 10, wherein the antibody heavy-chain variable region polypeptide includes a polypeptide consisting of the amino acid sequence represented by SEQ ID No:1 and the antibody light-chain variable region polypeptide includes a polypeptide consisting of the amino acid sequence represented by SEQ ID No:2.

12. The antigen-concentration measurement/detection method according to any one of Claims 7 to 10, wherein the antibody heavy-chain variable region polypeptide includes a polypeptide consisting of the amino acid sequence represented by SEQ ID No:6 and the antibody light-chain variable region polypeptide includes a polypeptide consisting of the amino acid sequence represented by SEQ ID No:7.

## Patentansprüche

1. Kit zur Messung/Erfassung einer Antigenkonzentration, umfassend ein Antikörperpolypeptid mit einer leichtkettigen variablen Region und ein Antikörperpolypeptid mit einer schwerkettigen variablen Region, von denen beide mit einem fluoreszierenden Farbstoff markiert sind, der in dem Zustand, wo er das Antikörperpolypeptid mit einer schwerkettigen variablen Region oder das Antikörperpolypeptid mit einer leichtkettigen variablen Region markiert, ausgelöscht wird, wobei die Auslöschung eliminiert wird, wenn das Antikörperpolypeptid mit einer schwerkettigen variablen Region und das Antikörperpolypeptid mit einer leichtkettigen variablen Region über ein Antigen einen Komplex bilden, um die Fluoreszenzintensität zu erhöhen, und wobei das Kit eine Messung der Antigenkonzentration oder Visualisierung des Antigens unter Bezugnahme auf eine positive Korrelation zwischen der Konzentration des Antigens in einer flüssigen Phase und der Fluoreszenzintensität des fluoreszierenden Farbstoffes ermöglicht.

2. Kit zur Messung/Erfassung einer Antigenkonzentration nach Anspruch 1, wobei das Antikörperpolypeptid mit einer schwerkettigen variablen Region und das Antikörperpolypeptid mit einer leichtkettigen variablen Region verbunden sind, um einen einkettigen Antikörper zu bilden.

3. Kit zur Messung/Erfassung einer Antigenkonzentration nach Anspruch 1 oder 2, wobei der fluoreszierende Farbstoff ein auf Rhodamin basierender fluoreszierender Farbstoff oder ein auf Oxazin basierender fluoreszierender Farbstoff ist.

4. Kit zur Messung/Erfassung einer Antigenkonzentration nach Anspruch 3, wobei der fluoreszierende Farbstoff CR110, TAMRA oder ATT0655 ist.

5. Kit zur Messung/Erfassung einer Antigenkonzentration nach einem der Ansprüche 1 bis 4, wobei das Antikörperpolypeptid mit einer schwerkettigen variablen Region ein Polypeptid umfasst, das aus der Aminosäuresequenz besteht, die durch SEQ ID Nr. 1 dargestellt ist, und das Antikörperpolypeptid mit einer leichtkettigen variablen Region ein Polypeptid umfasst, das aus der Aminosäuresequenz besteht, die durch SEQ ID Nr. 2 dargestellt ist.

6. Kit zur Messung/Erfassung einer Antigenkonzentration nach einem der Ansprüche 1 bis 4, wobei das Antikörperpolypeptid mit einer schwerkettigen variablen Region ein Polypeptid umfasst, das aus der Aminosäuresequenz besteht, die durch SEQ ID Nr. 6 dargestellt ist, und das Antikörperpolypeptid mit einer leichtkettigen variablen Region ein Polypeptid umfasst, das aus der Aminosäuresequenz besteht, die durch SEQ ID Nr. 7 dargestellt ist.

7. Verfahren zur Messung/Erfassung einer Antigenkonzentration, umfassend die folgenden Schritte (a) bis (c), fortlaufend in dieser Reihenfolge:
(a) In Kontakt Bringen eines Antikörperpolypeptids mit einer leichtkettigen variablen Region und eines Antikörperpolypeptids mit einer schwerkettigen variablen Region, die mit einem fluoreszierenden Farbstoff markiert sind, der in dem Zustand, wo er das Antikörperpolypeptid mit einer leichtkettigen variablen Region markiert, ausgelöscht wird; oder eines Antikörperpolypeptids mit einer schwerkettigen variablen Region und eines Antikörperpolypeptids mit einer leichtkettigen variablen Region, die mit einem fluoreszierenden Farbstoff markiert sind, der in dem Zustand, wo er das Antikörperpolypeptid mit einem schwerkettigen variablen Bereich markiert, ausgelöscht wird, mit
(a1) einem Antigen in einer Testsubstanz in einer liquiden Phase, oder
(a2) einem Antigen in einer Probe eines nicht menschlichen tierischen Testsubjektes, dem das Antikörperpolypeptid mit einer leichtkettigen variablen Region und das Antikörperpolypeptid mit einer schwerkettigen variablen Region, die mit einem fluoreszierenden Farbstoff markiert wurden; oder das Antikörperpolypeptid mit einer schwerkettigen variablen Region und das Antikörperpolypeptid mit einer leichtkettigen variablen Region, die mit einem fluoreszierenden Farbstoff markiert wurden, verabreicht wurde, oder
(a3) einem Antigen eines Testsubjektes in vitro;
(b) Messung der Fluoreszenzintensität des fluoreszierenden Farbstoffes im Falle von (a1) und Erfassen der Fluoreszenz des fluoreszierenden Farbstoffes im Falle von (a2) und (a3); und
(c) Erhalten des Niveaus an Antigen in der Testsubstanz im Falle von (a1) und sichtbar Machen des Antigens, das in dem Testsubjekt vorliegt in den Fällen (a2) und (a3), mittels eines Computers, unter Bezugnahme auf die positive Korrelation zwischen der Konzentration des Antigens in der liquiden Phase und der Fluoreszenzintensität des fluoreszierenden Farbstoffes, wobei der Auslöscher eliminiert wird, wenn das Antikörperpolypeptid mit einer schwerkettigen variablen Region und das Antikörperpolypeptid mit einer leichtkettigen variablen Region über das Antigen einen Komplex bilden, um die Fluoreszenzintensität zu erhöhen.

8. Verfahren zur Messung/Erfassung einer Antigenkonzentration nach Anspruch 7, wobei das Antikörperpolypeptid mit einer schwerkettigen variablen Region und das Antikörperpolypeptid mit einer leichtkettigen variablen Region verbunden sind, um einen einkettigen Antikörper zu bilden.

9. Verfahren zur Messung/Erfassung einer Antigenkonzentration nach Anspruch 7 oder 8, wobei der fluoreszierende Farbstoff ein auf Rhodamin basierender fluoreszierender Farbstoff oder ein auf Oxazin basierender fluoreszierender Farbstoff ist.

10. Verfahren zur Messung/Erfassung einer Antigenkonzentration nach Anspruch 9, wobei der fluoreszierende Farbstoff CR110, TAMRA oder ATT0655 ist.

11. Verfahren zur Messung/Erfassung einer Antigenkonzentration nach einem der Ansprüche 7 bis 10, wobei das Antikörperpolypeptid mit einer schwerkettigen variablen Region ein Polypeptid umfasst, das aus der Aminosäuresequenz besteht, die durch SEQ ID Nr. 1 dargestellt ist, und das Antikörperpolypeptid mit einer leichtkettigen variablen Region ein Polypeptid umfasst, das aus der Aminosäuresequenz besteht, die durch SEQ ID Nr. 2 dargestellt ist.

12. Verfahren zur Messung/Erfassung einer Antigenkonzentration nach einem der Ansprüche 7 bis 10, wobei das Antikörperpolypeptid mit einer schwerkettigen variablen Region ein Polypeptid umfasst, das aus der Aminosäuresequenz besteht, die durch SEQ ID Nr. 6 dargestellt ist, und das Antikörperpolypeptid mit einer leichtkettigen variablen Region ein Polypeptid umfasst, das aus der Aminosäuresequenz besteht, die durch SEQ ID Nr. 7 dargestellt ist.

## Revendications

1. Trousse pour la détection/mesure de la concentration en antigène, comprenant un polypeptide de la région variable de la chaîne légère d'un anticorps ainsi qu'un polypeptide de la région variable de la chaîne lourde d'un anticorps, dont l'un d'entre eux est marqué avec un colorant fluorescent qui est éteint dans l'état dans lequel il est marqué pour le polypeptide de la région variable de la chaîne lourde d'un anticorps ou le polypeptide de la région variable de la chaîne légère d'un anticorps, dans laquelle l'extincteur est éliminé quand le polypeptide de la région variable de la chaîne lourde d'un anticorps ainsi que le polypeptide de la région variable de la chaîne légère d'un anticorps forment un complexe, via un antigène, pour augmenter une intensité de fluorescence et dans laquelle la trousse permet d'obtenir une mesure de la concentration de l'antigène ou la visualisation de l'antigène, par référence à une corrélation positive existant entre la concentration de l'antigène, dans une phase liquide, et l'intensité de fluorescence dans le colorant fluorescent.

2. Trousse pour la détection/mesure de la concentration en antigène selon la revendication 1, dans laquelle le polypeptide de la région variable de la chaîne lourde d'un anticorps et le polypeptide de la région variable de la chaîne légère d'un anticorps sont liés pour former un anticorps en simple chaîne.

3. Trousse pour la détection/mesure de la concentration en antigène selon la revendication 1 ou la revendication 2, dans laquelle le colorant fluorescent est un colorant fluorescent à base de rhodamine ou un colorant fluorescent à base d'oxazine.

4. Trousse pour la détection/mesure de la concentration en antigène selon la revendication 3, dans laquelle le colorant fluorescent est le CR110, le TAMRA ou l'ATT0655.

5. Trousse pour la détection/mesure de la concentration en antigène selon l'une quelconque des revendications 1 à 4, dans laquelle le polypeptide de la région variable de la chaîne lourde d'un anticorps comprend un polypeptide constitué par la séquence d'acides aminés représentée par la SEQ ID n° : 1 et le polypeptide de la région variable de la chaîne légère d'un anticorps comprend un polypeptide constitué par la séquence d'acides aminés représentée par la SEQ ID n° : 2.

6. Trousse pour la détection/mesure de la concentration en antigène selon l'une quelconque des revendications 1 à 4, dans laquelle le polypeptide de la région variable de la chaîne lourde d'un anticorps comprend un polypeptide constitué par la séquence d'acides aminés représentée par la SEQ ID n° : 6 et le polypeptide de la région variable de la chaîne légère d'un anticorps comprend un polypeptide constitué par la séquence d'acides aminés représentée par la SEQ ID n° : 7.

7. Procédé de détection/mesure de la concentration en antigène comprenant les étapes (a) à (c) suivantes, séquentiellement dans cet ordre :
(a) mettre en contact un polypeptide de la région variable de la chaîne légère d'un anticorps et un polypeptide de la région variable de la chaîne lourde d'un anticorps, marqué avec un colorant fluorescent qui est éteint dans l'état dans lequel il est marqué pour le polypeptide de la région variable de la chaîne légère d'un anticorps ; ou un polypeptide de la région variable de la chaîne lourde d'un anticorps et un polypeptide de la région variable de la chaîne légère d'un anticorps, marqué avec un colorant fluorescent qui est éteint dans l'état dans lequel il est marqué pour le polypeptide de la région variable de la chaîne lourde d'un anticorps, avec :
(a1) un antigène dans une substance à tester, dans une phase liquide, ou
(a2) un antigène, dans un échantillon d'un sujet animal à tester n'étant pas humain à qui on a administré le polypeptide de la région variable de la chaîne légère d'un anticorps ainsi que le polypeptide de la région variable de la chaîne lourde d'un anticorps marqué avec un colorant fluorescent ; ou le polypeptide de la région variable de la chaîne lourde d'un anticorps et le polypeptide de la région variable de la chaîne légère d'un anticorps marqué avec un colorant fluorescent, ou
(a3) un antigène d'un sujet à tester in vitro ;
(b) mesurer l'intensité de fluorescence du colorant fluorescent, dans le cas cité en (a1), et détecter la fluorescence du colorant fluorescent dans les cas cités en (a2) et (a3) ; et
(c) obtenir, au moyen de l'informatique, le taux de l'antigène contenu dans la substance à tester, dans le cas cité en (a1), et visualiser l'antigène contenu dans le sujet à tester dans les cas cités en (a2) et (a3), par référence à la corrélation positive existant entre la concentration de l'antigène, dans la phase liquide, et l'intensité de fluorescence du colorant fluorescent, dans laquelle l'extincteur est éliminé quand le polypeptide de la région variable de la chaîne lourde d'un anticorps ainsi que le polypeptide de la région variable de la chaîne légère d'un anticorps forment un complexe, via l'antigène, pour augmenter l'intensité de fluorescence.

8. Procédé de détection/mesure de la concentration en antigène selon la revendication 7, dans lequel le polypeptide de la région variable de la chaîne lourde d'un anticorps ainsi que le polypeptide de la région variable de la chaîne légère d'un anticorps sont liés pour former un anticorps en chaîne simple.

9. Procédé de détection/mesure de la concentration en antigène selon la revendication 7 ou la revendication 8, dans lequel le colorant fluorescent est un colorant fluorescent à base de rhodamine ou un colorant fluorescent à base d'oxazine.

10. Procédé de détection/mesure de la concentration en antigène selon la revendication 9, dans lequel le colorant fluorescent est le CR110, le TAMRA ou l'ATT0655.

11. Procédé de détection/mesure de la concentration en antigène selon l'une quelconque des revendications 7 à 10, dans lequel le polypeptide de la région variable de la chaîne lourde d'un anticorps comprend un polypeptide constitué par la séquence d'acides aminés représentée par la SEQ ID n° : 1 et le polypeptide de la région variable de la chaîne légère d'un anticorps comprend un polypeptide constitué par la séquence d'acides aminés représentée par la SEQ ID n° : 2.

12. Procédé de détection/mesure de la concentration en antigène selon l'une quelconque des revendications 7 à 10, dans laquelle le polypeptide de la région variable de la chaîne lourde d'un anticorps comprend un polypeptide constitué par la séquence d'acides aminés représentée par la SEQ ID n° : 6 et le polypeptide de la région variable de la chaîne légère d'un anticorps comprend un polypeptide constitué par la séquence d'acides aminés représentée par la SEQ ID n° : 7.
